# EUROPEAN PATENT SPECIFICATION

(11) **EP 3 271 479 B1**
(45) Date of publication and mention of the grant of the patent: **17.07.2019**
(21) Application number: 16722414.6
(22) Date of filing: 17.03.2016
(51) Int. Cl.: C12Q 1/68

(54) **METHODS AND MEANS FOR SUBTYPING INVASIVE LOBULAR BREAST CANCER**
VERFAHREN UND MITTEL ZUR UNTERTYPISIERUNG VON INVASIVEM LOBULÄREM BRUSTKREBS
PROCÉDÉS ET MOYENS POUR LE SOUS-TYPAGE DE CANCER DU SEIN LOBULAIRE INVASIF

(30) Priority: 17.03.2015 EP 15159513
(43) Date of publication of application: 24.01.2018
(73) Proprietor: Stichting Het Nederlands Kanker Instituut- Antoni van Leeuwenhoek Ziekenhuis, 1066 CX Amsterdam (NL); Agendia N.V., 1098 XH Amsterdam (NL)
(72) Inventor: BISMEIJER, Tycho, 1066 CX Amsterdam (NL); MICHAUT, Magali, 1066 CX Amsterdam (NL); BERNARDS, Rene, 1066 CX Amsterdam (NL); WESSELS, Lodewyk Frederik Ary, 1066 CX Amsterdam (NL)
(74) Representative: V.O.
(86) International application number: PCT/NL2016/050191
(87) International publication number: WO 2016/148573

(56) References cited:
- YAN XU ET AL: "The application of gene co-expression network reconstruction based on CNVs and gene expression microarray data in breast cancer", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 2, 25 May 2011 (2011-05-25), pages 1627-1637, XP019992203, ISSN: 1573-4978, DOI: 10.1007/S11033-011-0902-3 -& Yan Xu ET AL: "The application of gene co-expression network reconstruction based on CNVs and gene expression microarray data in breast cancer - Supplementary Material", Molecular Biology Reports, 25 May 2011 (2011-05-25), XP055226259, Retrieved from the Internet: URL:http://link.springer.com/content/esm/a rt:10.1007/s11033-011-0902-3/file/MediaObj ects/11033_2011_902_MOESM3_ESM.pdf [retrieved on 2015-11-05]
- F BERTUCCI ET AL: "Lobular and ductal carcinomas of the breast have distinct genomic and expression profiles", ONCOGENE, vol. 27, no. 40, 19 May 2008 (2008-05-19), pages 5359-5372, XP055226288, GB ISSN: 0950-9232, DOI: 10.1038/onc.2008.158 cited in the application

## Description

### The invention

Breast cancer is a heterogeneous disease and has traditionally been subdivided into distinct histological subtypes based on cell morphology. About 60-75% of breast cancers are invasive ductal carcinomas (IDC) (Guiu et al., 2014. Critical Rev Oncol/Hematol 92: 235-257). The next most common subtype is invasive lobular carcinoma (ILC), representing 5-15% of all breast cancers (Guiu et al., 2014. Critical Rev Oncol/Hematol 92: 235-257; Pestalozzi et al., 2008. J Clin Oncol 26: 3006-3014). ILC can be subdivided into five more specific histological subtypes (Iorfida et al., 2012. Breast Cancer Res Treatment 133: 713-723). ILCs are typically estrogen receptor (ER) and/or progesterone (PR) positive and exhibit frequent loss of the cellular adhesion molecule E-cadherin (CDH1) expression (Guiu et al., 2014. Critical Rev Oncol/Hematol 92: 235-257). A subset of ILCs is HER2 positive. ILCs have very similar survival to IDCs at both five and 10 years, but despite this similar survival, the clinical course is distinct: ILCs are three times more likely to metastasize to the peritoneum, gastrointestinal tract, and ovaries and are more frequently bilateral (Arpino et al., 2004. BCR 6: R149-156).

Gene expression-based molecular subtypes have been used as a reference to describe breast cancers (Perou et al., 2000. Nature 406: 747-752; Sørlie et al., 2003. PNAS USA 100: 8418-8423; Xu et al., 2012. Mol Biol Reports 39: 1627-1637). Such subtypes are relatively well reflected in the immunohistochemistry (IHC)-based diagnosis used in the clinic (de Ronde et al., 2010. Breast Can Res Treatment 119: 119-126). However, they were mainly defined based on IDCs. Some molecular studies have been performed on ILC, using comparative genomic hybridization (Weigelt B, et al., 2010. J Pathol 220: 45-57) or gene expression profiling (Bertucci et al., 2008. Oncogene 27: 5359-5372), and more recently targeted DNA sequencing in advanced disease (Ross et al., 2013. Clin Canc Res: 19: 2668-2676). Two recent studies extensively characterizing large breast cancer cohorts (Curtis et al., 2012. Nature 486: 346-352; The Cancer Genome Atlas, 2012. Nature 490: 61-7011) contain ILCs, but are dominated by IDCs, leaving ILC largely uncharacterized.

Treatment decisions made by oncologists for breast cancer are mainly based on results obtained in large trials, in which ILCs are only a minor subgroup. It is, therefore, not always the case that the conclusions from "breast cancer" trials also apply to ILC. For instance, ILCs were found to be less responsive to neo-adjuvant chemotherapy than IDCs, but this can possibly be explained by differences in molecular characteristics, particularly hormonal status and HER2 expression (Lips et al., 2012. Breast Canc Res Treatment 136: 35-43). In general, evidence that ILCs have a similar or worse clinical outcome as compared to IDCs are not robust (Guiu et al., 2014. Critical Rev Oncol/Hematol 92: 235-257), but the distinct pattern of distant metastases indicates that ILCs have unique biology.

There is thus a need for typing ILCs to identify subtypes that are more aggressive and life-threatening, and which should be treated with aggressive treatments. In addition, knowledge about subtypes may help to identify subtypes that are more sensitive to a drug or treatment regimen than others.

The invention therefore provides a method of typing a sample from an invasive lobular breast cancer patient, the method comprising providing a sample comprising RNA expression products from breast cancer cells from the patient, determining a level of RNA expression for at least two genes that are selected from Table 1 comprising at least GIMAP6 and P2RY14 in said sample, comparing said determined level of RNA expression of the at least two genes to the level of expression of the genes in a reference sample; and typing said sample based on the comparison of the determined levels of RNA expression.

A sample comprising RNA expression products from a breast cancer cell of a patient is preferably provided after the removal of all or part of a breast cancer sample from the patient during surgery biopsy. For example, a sample comprising RNA may be obtained from a needle biopsy sample or from a tissue sample comprising breast cancer cells that was previously removed by surgery. The surgical step of removing a relevant tissue sample, in this case a breast cancer sample, from an individual is not part of a method according to the invention.

A sample from a breast cancer patient comprising RNA expression products from a tumor of the patient can be obtained in numerous ways, as is known to a skilled person. For example, the sample can be freshly prepared from cells or a tissue sample at the moment of harvesting, or it can be prepared from samples that are stored at -70°C until processed for sample preparation. Alternatively, tissues or biopsies can be stored under conditions that preserve the quality of the protein or RNA. Examples of these preservative conditions are fixation using e.g. formaline and paraffin embedding, RNase inhibitors such as RNAsin® (Pharmingen) or RNasecure® (Ambion), aquous solutions such as RNAlater® (Assuragen; US06204375), Hepes-Glutamic acid buffer mediated Organic solvent Protection Effect (HOPE; DE10021390), and RCL2 (Alphelys; WO04083369), and non-aquous solutions such as Universal Molecular Fixative (Sakura Finetek USA Inc.; US7138226).

RNA may be isolated from a breast tissue sample comprising breast cancer cells by any technique known in the art, including but not limited to Trizol (Invitrogen; Carlsbad, California), RNAqueous® (Applied Biosystems/Ambion, Austin, Tx), Qiazol® (Qiagen, Hilden, Germany), Agilent Total RNA Isolation Lits (Agilent; Santa Clara, California), RNA-Bee® (Tel-Test. Friendswood, Texas), and Maxwell™ 16 Total RNA Purification Kit (Promega; Madison, Wisconsin). A preferred RNA isolation procedure involves the use of Qiazol® (Qiagen, Hilden, Germany). RNA can be extracted from a whole sample or from a portion of a sample generated by, for example section or laser dissection.

The level of RNA expression of a gene of Table 1 can be determined by any method known in the art. Methods to determine RNA levels of genes are known to a skilled person and include, but are not limited to, Northern blotting, quantitative Polymerase chain reaction (qPCR), also termed real time PCR (rtPCR), microarray analysis and next generation RNA sequencing. The term qPCR refers to a method that allows amplification of relatively short (usually 100 to 1000 basepairs) of DNA sequences. In order to measure messenger RNA (mRNA), the method is extended using reverse transcriptase to convert mRNA into complementary DNA (cDNA) which is then amplified by PCR. The amount of product that is amplified can be quantified using, for example, TaqMan® (Applied Biosystems, Foster City, CA, USA), Molecular Beacons, Scorpions® and SYBR® Green (Molecular Probes). Quantitative Nucleic acid sequence based amplification (qNASBA) can be used as an alternative for qPCR.

A preferred method for determining a level of RNA expression is microarray analysis. For microarray analysis, a hybridization mixture is prepared by extracting and labelling of RNA. The extracted RNA is preferably converted into a labelled sample comprising either complementary DNA (cDNA) or cRNA using a reverse-transcriptase enzyme and labelled nucleotides. A preferred labelling introduces fluorescently-labelled nucleotides such as, but not limited to, cyanine-3-CTP or cyanine-5-CTP. Examples of labelling methods are known in the art and include Low RNA Input Fluorescent Labelling Kit (Agilent Technologies), MessageAmp Kit (Ambion) and Microarray Labelling Kit (Stratagene).

A labelled sample preferably comprises two dyes that are used in a so-called two-colour array. For this, the sample is split in two or more parts, and one of the parts is labelled with a first fluorescent dye, while a second part is labelled with a second fluorescent dye. The labelled first part and the labelled second part are independently hybridized to a microarray. The duplicate hybridizations with the same samples allow compensating for dye bias.

More preferably, a sample from a breast cancer patient is labelled with a first fluorescent dye, while a reference sample, for example a sample from a breast cancer pool or a sample from a relevant cell line or mixture of cell lines, is labelled with a second fluorescent dye. The labelled sample and the labelled reference are co-hybridized to a microarray. Even more preferred, a sample is labelled with a fluorescent dye and hybridized to a microarray without a reference sample.

The labelled sample can be hybridized against the probe molecules that are spotted on the array. A molecule in the labelled sample will bind to its appropriate complementary target sequence on the array. Before hybridization, the arrays are preferably incubated at high temperature with solutions of saline-sodium buffer (SSC), Sodium Dodecyl Sulfate (SDS) and bovine serum albumin (BSA) to reduce background due to nonspecific binding.

The arrays are preferably washed after hybridization to remove labelled sample that did not hybridize on the array, and to increase stringency of the experiment by reducing cross hybridization of the labelled sample to a partial complementary probe sequence on the array. An increased stringency will substantially reduce non-specific hybridization of the sample, while specific hybridization of the sample is not substantially reduced. Stringent conditions include, for example, washing steps for five minutes at room temperature 0.1x Sodium chloride-Sodium Citrate buffer (SSC)/0.005% Triton X-102. More stringent conditions include washing steps at elevated temperatures, such as 37 degrees Celsius, 45 degrees Celsius, or 65 degrees Celsius, either or not combined with a reduction in ionic strength of the buffer to 0,05x SSC or 0,01x SSC as is known to a skilled person.

Image acquisition and data analysis can subsequently be performed to produce an image of the surface of the hybridised array. For this, the slide can be dried and placed into a laser scanner to determine the amount of labelled sample that is bound to a target spot. Laser excitation yields an emission with characteristic spectra that is indicative of the labelled sample that is hybridized to a probe molecule. In addition, the amount of labelled sample can be quantified.

The level of expression, preferably mRNA expression levels of genes depicted in Table 1, is preferably compared to levels of expression of the same genes in a reference sample. A reference sample is preferably an RNA sample isolated from a tissue of a healthy individual, preferably comprising breast cells. A preferred reference sample comprises a RNA sample from a relevant cell line or mixture of cell lines. The RNA from a cell line or cell line mixture can be produced in-house or obtained from a commercial source such as, for example, Stratagene Human Reference RNA. A further preferred reference sample comprises RNA isolated and pooled from normal adjacent tissue from cancer patients, preferably breast cancer patients.

A more preferred reference sample comprises an RNA sample from an individual suffering from breast cancer, more preferred from multiple individuals suffering from breast cancer. It is preferred that said multiple samples are pooled from more than 10 individuals, more preferred more than 20 individuals, more preferred more than 30 individuals, more preferred more than 40 individuals, most preferred more than 50 individuals. A most preferred reference sample comprises a pooled RNA sample that is isolated from tissue comprising breast cancer cells from multiple individuals suffering from breast cancer.

As an alternative, a static reference can be generated which enables performing single channel hybridizations for this test. A preferred static reference is calculated by measuring the median background-subtracted level of expression (rMeanSignal) of a gene across 5 hybridizations of a reference sample, preferably obtained from pooled breast cancer samples, on a microarray. The level of expression may be normalized as is known a skilled person. Subsequently, log-ratios for each gene/probe hybridization is generated relative to the value of the static reference.

Typing of a sample can be performed in various ways. In one method, a coefficient is determined that is a measure of a similarity or dissimilarity of a sample with said reference sample. A number of different coefficients can be used for determining a correlation between the RNA expression level in an RNA sample from an individual and a reference sample. Preferred methods are parametric methods which assume a normal distribution of the data.

The result of a comparison of the determined expression levels with the expression levels of the same genes in at least one reference sample is preferably displayed or outputted to a user interface device, a computer readable storage medium, or a local or remote computer system. The storage medium may include, but is not limited to, a floppy disk, an optical disk, a compact disk read-only memory (CD-ROM), a compact disk rewritable (CD-RW), a memory stick, and a magneto-optical disk.

The expression data are preferably normalized. Normalization refers to a method for adjusting or correcting a systematic error in the measurements of detected label. Systemic bias results in variation by inter-array differences in overall performance, which can be due to for example inconsistencies in array fabrication, staining and scanning, and variation between labeled RNA samples, which can be due for example to variations in purity. Systemic bias can be introduced during the handling of the sample in a microarray experiment.

To reduce systemic bias, the determined RNA levels are preferably corrected for background non-specific hybridization and normalized using, for example, Feature Extraction software (Agilent Technologies). Other methods that are or will be known to a person of ordinary skill in the art, such as a dye swap experiment (Martin-Magniette et al., Bioinformatics 21:1995-2000 (2005)) can also be applied to normalize differences introduced by dye bias. Normalization of the expression levels results in normalized expression values.

Conventional methods for normalization of array data include global analysis, which is based on the assumption that the majority of genetic markers on an array are not differentially expressed between samples [Yang et al., Nucl Acids Res 30:15 (2002)]. Alternatively, the array may comprise specific probes that are used for normalization. These probes preferably detect RNA products from housekeeping genes such as glyceraldehyde-3-phosphate dehydrogenase and 18S rRNA levels, of which the RNA level is thought to be constant in a given cell and independent from the developmental stage or prognosis of said cell.

Therefore, a preferred method according to the invention further comprises normalizing the determined RNA levels of said at least two genes listed in Table 1, comprising at least GIMAP6 and P2RY14, in said sample.

Said normalization preferably comprises median centering, in which the "centers" of the array data are brought to the same level under the assumption that the majority of genes are not changed between conditions. Said normalization preferably comprises Lowess (LOcally WEighted Scatterplot Smoothing) local regression normalization to correct for both print-tip and intensity-dependent bias.

In a preferred embodiment, genes are selected of which the RNA expression levels are largely constant between individual tissue samples comprising cancer cells from one individual, and between tissue samples comprising cancer cells from different individuals. It will be clear to a skilled artisan that the RNA levels of said set of normalization genes preferably allow normalization over the whole range of RNA levels. An example of a set of normalization genes is provided in WO 2008/039071.

The sample from a breast cancer patient comprises invasive lobular breast cancer cells.

Breast carcinomas are divided into 2 main types, ductal and lobular, based on their phenotypic appearance. Invasive ductal carcinoma (IDC) is the most common type of breast cancer, which originates in a duct. Invasive lobular carcinoma (ILC) accounts for about 10% of all breast cancers and originates in a lobule. Methods and means for differentiating between IDC and ILC are known in the art. For example, classical ILC is a low-grade tumor with little or no nuclear atypia and a low mitotic rate. ILCs are characterized by cytologically uniform cells with round nuclei and inconspicuous nucleoli, as well as discohesive architecture with a linear or non-linear growth pattern (Martinez and Azzopardi, 1979.Histopathology 3: 467-488; Dixon et al., 1982. Histopathology 6: 149-161).

In addition, E-cadherin is differentially expressed in lobular and ductal breast cancers. Membrane expression of E-cadherin is present in invasive and in situ ductal carcinomas, while almost all lobular carcinomas showed complete loss of expression of E-cadherin (Acs et al., 2001. Am J Clin Pathol 115: 85-98).

Therefore, routine pathology, including immuno-histochemical staining for E-cadherin, enables to determine whether a sample from a breast cancer patient comprises invasive lobular breast cancer cells, or invasive

A method for typing breast cancer cells according to the invention preferably further comprises determining the presence of a mutation in the GATA3 gene and/or in the ERBB2 gene.

The term "GATA3", as used herein, refers to a gene on human chromosome 10 that is also known as GATA Binding Protein 3, and GATA-Binding Factor 3. GATA3 encodes a transcription factor. Database references for GATA3 include Entrez Gene Number 2625 and Ensembl Number ENSG00000107485. The gene encodes four alternatively spliced mRNA molecules, having Reference Sequence (RefSeq) numbers NM_001002295.1, NM_002051.2, XM_005252442.1 and XM_005252443.2.

The term "ERBB2", as used herein, refers to a gene on human chromosome 17 that is also termed proto-oncogene C-ErbB-2, neuroblastoma/glioblastoma derived oncogene homolog 2, and proto-oncogene Neu. ERBB2 encodes a receptor tyrosine kinase. Database references for ERBB2 include Entrez Gene Number 2064 and Ensembl Number ENSG00000141736. The gene encodes ten alternatively spliced mRNA molecules, having Reference Sequence (RefSeq) numbers NM_001005862.2, NM_001289936.1, NM_001289937.1, NM_001289938.1, NM_004448.3., XM_005257140.1, XM_006721765.1, XM_006721766.1, XM_006721767.1, and XM_006721768.1.

The presence of a mutation in the GATA3 and/or in the ERBB2 gene is preferably determined by comparing a sequence of the GATA3 gene and/or the ERBB2 gene that is obtained from a breast cancer cell with a reference sequence. A reference sequence of GATA3 and/or ERBB2 corresponds to the wild type nucleotide sequence of GATA3 and/or ERBB2. As a model for the wild type sequences, reference is made to the indicated RefSeq numbers. However, a reference can also be derived from a non-affected individual such as, for example, a non-affected close relative.

A nucleotide sequence of GATA3 and/or ERBB2 that is mutated or altered compared to a nucleotide sequence of GATA3 and/or ERBB2 in a reference sample preferably refers to an altered nucleotide sequence that results in an alteration of the amino acid sequence of the encoded protein.

A nucleotide sequence of GATA3 and/or ERBB2 can be determined by any method known in the art, including but not limited to sequence analysis of a genomic region encoding GATA3 and/or ERBB2; sequence analysis of a mRNA product or a derivative of a mRNA product such as a cDNA product, of GATA3 and/or ERBB2.

Sequence determination can be performed by any method known in the art, including but not limited to dideoxy sequencing, matrix-assisted laser desorption/ionization time-of-flight mass spectrometry, and sequencing by hybridization, including hybridization with sequence-specific oligonucleotides and hybridization to oligonucleotide arrays. The nucleotide sequence of GATA3 and/or ERBB2 can also be determined by application of mutation analysis methods such as single stranded conformation polymorphism, DNA heteroduplex analysis, denaturing gradient gel electrophoresis and thermal gradient gel electrophoresis.

Sequence analyses can be performed either by direct sequence analysis of a relevant nucleic acid molecule comprising the GATA3 gene and/or ERBB2 gene, or a mRNA product thereof, or by indirect sequencing of a relevant nucleic acid after amplification of all or any part of the GATA3 and/or ERBB2 gene or a mRNA product thereof. Alternative direct or indirect methods comprise hybridization protection assay, allele-specific amplification, ligase-mediated detection, primer extension, and restriction fragment length analysis.

In an alternative embodiment, the presence of a mutation in GATA3 and/or ERBB2 can be determined by analysis of the encoded protein by, for example, protein sequence determination, two dimensional gel electrophoresis, multidimensional protein identification technology, ELISA, liquid chromatography-mass spectrometry (LC-MS), matrix-assisted laser desorption/ionization time-of-flight mass spectrometry (MALDI-TOF), or the use of antibodies that interact with either a non-mutated wild type form of GATA3 or ERBB2, or with a mutated variant form of GATA3 or ERBB2.

A method for typing breast cancer cells according to the invention preferably further comprises determining a level of expression of PDL1, PD1, and/or CTLA4.

The term "PD1", as used herein, refers to a gene on human chromosome 2 that is also termed CD279, Programmed Cell Death 1, PDCD1 and Systemic Lupus Erythematosus Susceptibility 2. PD1 encodes a cell surface membrane protein. Database references for PDL1 include Entrez Gene Number 5133 and Ensembl Number ENSG00000188389. The gene encodes three alternatively spliced mRNA molecules, having Reference Sequence (RefSeq) numbers NM_005018.2, XM_006712573.1, and XM_006724986.1.

The term "PDL1", as used herein, refers to a gene on human chromosome 9 that is also termed CD274, Programmed Cell Death 1 ligand 1 and B7 homolog 1. PDL1 encodes a type I transmembrane protein. Database references for PDL1 include Entrez Gene Number 29126 and Ensembl Number ENSG00000120217. The gene encodes three alternatively spliced mRNA molecules, having Reference Sequence (RefSeq) numbers NM_001267706.1, NM_014143.3, and XM_006716759.1.

The term "CTLA4", as used herein, refers to a gene on human chromosome 2 that is also termed Cytotoxic T-Lymphocyte-Associated Protein 4, CD2, CELIAC3, GSE, and CD152. CTLA4 encodes a member of the immunoglobulin superfamily. Database references for CTLA4 include Entrez Gene Number 1493 and Ensembl Number ENSG00000163599. The gene encodes two alternatively spliced mRNA molecules, having Reference Sequence (RefSeq) numbers NM_001037631.2 and NM_005214.4.

Methods to determine a level of expression of PDL1, PD1, and/or CTLA4 include two dimensional gel electrophoresis, multidimensional protein identification technology, ELISA, liquid chromatography-mass spectrometry (LC-MS), matrix-assisted laser desorption/ionization time-of-flight mass spectrometry (MALDI-TOF), and the *in situ* use of antibodies that specifically interact with PDL1, PD1, and/or CTLA4. Said antibodies are preferably directly or indirectly labeled with a detectable label, for example a fluorescent or radioactive label. The term "indirect labeling" is known to the skilled person and includes the use of labeled secondary antibodies that react with the antibodies that specifically interact with PDL1, PD1, and/or CTLA4 , and/or the use of biotin-streptavidin interactions.

The level of expression of expression of PDL1, PD1, and/or CTLA4 is preferably compared to a level of expression in a reference, which preferably is sample isolated from a tissue of a healthy individual, preferably comprising breast cells. A more preferred reference comprises protein expression products from an individual suffering from breast cancer, more preferred from multiple individuals suffering from breast cancer. It is preferred that said multiple samples are pooled from more than 10 individuals, more preferred more than 20 individuals, more preferred more than 30 individuals, more preferred more than 40 individuals, most preferred more than 50 individuals. As an alternative, a static reference can be generated which enables performing single channel determinations such as ELISA for this test. The level of expression may be normalized as is known a skilled person.

Said at least two genes that are selected from Table 1 comprise GTPase, IMAP Family Member 6 (GIMAP6), also termed Immune Associated Nucleotide 2, Immune Associated Nucleotide 6, Entrez Gene: 474344, and Purinergic Receptor P2Y, G-Protein Coupled, 14 (P2RY14), also termed BPR105, GPR105, or KIAA0001, Entrez Gene: 9934. It was found that an increase of expression of GIMAP6 and P2RY14 in a sample comprising expression products of a breast cancer cell, when compared to an average level of expression of GIMAP6 and P2RY14 in ILCs, is indicative of an Immune Related (IR) subtype.

Said at least two genes that are selected from Table 1 preferably comprise GIMAP6, P2RY14 and CD79B (CD79b Molecule, Immunoglobulin-Associated Beta, Ig-beta, IGB, B-Cell-Specific Glycoprotein B29, Entrez Gene: 974); more preferably GIMAP6, P2RY14, CD79B and GIMAP5 (GTPase, IMAP Family Member 5, IROD, IAN4L1, IMAP3, Immune Associated Nucleotide 4 Like 1, Entrez Gene: 55340), more preferably GIMAP6, P2RY14, CD79B, GIMAP5 and GIMAP8 (GTPase, IMAP Family Member 8, Immune-Associated Nucleotide-Binding Protein 9, Human Immune Associated Nucleotide 6, Entrez Gene: 155038), more preferably GIMAP6, P2RY14, CD79B, GIMAP5, GIMAP8 and LOC100290115 (Entrez Gene: 100290115), more preferably GIMAP6, P2RY14, CD79B, GIMAP5, GIMAP8, LOC100290115 and IL33 (Interleukin 33, Nuclear Factor For High Endothelial Venules (NF-HEV), C9orf26, DVS27-Related Protein), more preferably GIMAP6, P2RY14, CD79B, GIMAP5, GIMAP8, LOC100290115, IL33 and SEL1L3 (Sel-1 Suppressor Of Lin-12-Like 3, Suppressor Of Lin-12-Like Protein 3, KIAA0746 Protein1, Entrez Gene: 23231), more preferably GIMAP6, P2RY14, CD79B, GIMAP5, GIMAP8, LOC100290115, IL33, SEL1L3 and SLC9A9 (Solute Carrier Family 9, Subfamily A (NHE9, Cation Proton Antiporter 9), Member 9, Putative Protein Product Of Nbla00118, NHE9, Sodium/Hydrogen Exchanger 9, Entrez Gene: 285195).

Further genes preferably include B4GALNT4 (Beta-1,4-N-Acetyl-Galactosaminyl Transferase 4, betaGT4, Beta-1,4-N-Acetylgalactosaminyltransferase IV, Entrez Gene: 338707), more preferably B4GALNT4 and TTLL12 (Tubulin Tyrosine Ligase-Like Family, Member 12, dJ526I14.2, KIAA0153, Entrez Gene: 23170), more preferably B4GALNT4, TTLL12 and NKAIN1 (Na+/K+ Transporting ATPase Interacting 1, FAM77C, Family With Sequence Similarity 77, Member C, Na(+)/K(+)-Transporting ATPase Subunit Beta-1-Interacting Protein 1, Entrez Gene: 79570), more preferably B4GALNT4, TTLL12, NKAIN1 and MMP11 (Matrix Metallopeptidase 11, stromelysin 3, Entrez Gene: 4320). A decrease of expression of B4GALNT4, TTLL12, NKAIN1 and MMP11 in a sample comprising expression products of a breast cancer cell, when compared to an average level of expression in ILC, is indicative of an Immune Related (IR) subtype.

In a further preferred method according to the invention, a level of RNA expression of at least five genes from Table 1, comprising at least GIMAP6 and P2RY14, is determined.

In a further preferred method according to invention, a level of RNA expression of all genes from Table 1 is determined.

A preferred method according to the invention further comprises determining a metastasizing potential of the sample from the patient. Methods of determining a metastasizing potential of a sample comprising breast cancer cells are known in the art and include, for example, a 70 gene Amsterdam profile (MammaPrint ®; (van 't Veer et al., 2002. Nature 415: 530) and other multigene expression tests such as a 21 gene signature (Oncotype DX®; Paik et al., 2004. New Engl J Med 351: 2817) and EndoPredict (Filipits et al., 2011. Clinical Cancer Research 17: 6012). A preferred method of determining a metastasizing potential is the 70 gene Amsterdam profile.

Using the methods of the invention, the presence of mutations in GATA3, loss of part or all of the long arm of chromosome 1, and enhanced expression of PDL1, PD1 and CTLA4, when compared to a reference, were found to be characteristic for an Immune Related (IR) subtype of invasive lobular breast cancer cells. In contrast, the presence of mutations in ERBB2, gain of part or all of the long arm of chromosome 8 and loss of part or all of the long arm of chromosome 11, and enhanced expression of progesterone receptor, estrogen receptor, GATA3, and fibronectin 1 (FN1), when compared to a reference, were found to be characteristic for an Hormone Response (HR) subtype of invasive lobular breast cancer cells.

Methods for determining a level of expression of progesterone receptor (PR), estrogen receptor (ER) and fibronectin 1 (FN1) are known in the art, including IHC, determination of mRNA levels, and the use of gene profiles that are indicative of the activity of PR and/or ER, for example as is described in the published US patent application US20130178381.

The analyses indicated herein above resulted in the identification of an Immune Related (IR) subtype for about 2/3 of all invasive lobular breast cancer cells, and an Hormone Response (HR) subtype for about 1/3 of all invasive lobular breast cancer cells.

Based on this classification, the method according to the invention may further comprise the determination of a strategy for treatment of the patient.

The invention therefore provides a method of assigning treatment to a patient suffering from breast cancer, comprising (a) typing a sample comprising RNA expression products from breast cancer cells from the patient according to a method of the invention, (b) classifying said sample as Immune Related (IR) or Hormone Response (HR), and (c) assigning anti-Estrogen Receptor directed therapy to a patient of which the sample is classified as HR.

Enhanced expression of ER in cancer cells of the HR subtype may be indicative for applying anti-estrogen receptor-directed therapy (anti-ER), for example comprising a selective estrogen receptor modulator (SERM) such as tamoxifen to said breast cancer patient. Tamoxifen and tamoxifen derivatives such as toremifene, are known antagonistic compounds of the estrogen receptor. Tamoxifen may be administered at 20 to 200 mg/kg per day, for example as Tamoxifen Citrate Tablets USP for oral administration. Toremifene similarly can be administered as toremifene citrate at 10 to 800 mg/d orally.

Further preferred SERMs include fulvestrant (7α,17β)-7-{9-[(4,4,5,5,5-pentafluoropentyl)sulfinyl]nonyl}estra-1,3,5(10)-triene-3,17-diol), which is an estrogen receptor antagonist with no agonist effects, which works by down-regulating the estrogen receptor. It is administered as a once-monthly injection at 500 mg; raloxifene ([6-hydroxy-2-(4-hydroxyphenyl)- benzothiophen-3-yl]- [4-[2-(1-piperidyl)ethoxy]phenyl]-methanone). It is an estrogen receptor antagonist in breast cells, including breast cancer cells. It can be orally administered at 60-240 mg/kg/day; and lasofoxifene ((5R,6S)-6-phenyl-5-[4-(2-pyrrolidin-1-ylethoxy)phenyl]-5,6,7,8-tetrahydronaphthalen-2-ol). It is an estrogen receptor antagonist in breast cells, including breast cancer cells. It can be orally administered at 0.001 mg/kg - 1.0 mg/kg/day.

A further preferred anti-ER directed therapy comprises the administration of an aromatase inhibitor. These non-steroidal inhibitors inhibit the synthesis of estrogen via reversible competition for the aromatase enzyme. Preferred aromatase inhibitors include anastrozole (2,2'-[5-(1H-1,2,4-triazol-1-ylmethyl)-1,3-phenylene]bis(2-methylpropanenitrile) and exemestane (6-Methylideneandrosta-1,4-diene-3,17-dione). Anastrozole can be orally administered at 1.0 - 10 mg/day. Exemestane can be orally administered at 25-50 mg/day.

The invention therefore provides a method of assigning treatment to a patient suffering from breast cancer, comprising (a) typing a sample comprising RNA expression products from breast cancer cells from the patient according to a method of the invention, (b) classifying said sample as Immune Related (IR) or Hormone Response (HR), and (c) assigning therapy targeting PD1, PDL1 and/or CTLA4 and/or assigning therapy comprising at least one DNA damaging agent to a patient of which the sample is classified as IR.

The programmed cell death 1 (PD1) receptor is an inhibitory receptor expressed by T cells following prolonged exposure to antigens. Its primary ligand, PDL1 is often expressed within the tumor microenvironment, including IR invasive lobular breast cancer cells. The PD1 receptor has a second ligand, PDL2 (also known as B7-DC or CD273), that is preferentially expressed by antigen-presenting cells. PD1 negatively regulates the effector phase of T-cell responses after ligation of PDL1 expressed within the tumor. Manipulation of the immune checkpoints, which include PD1, PDL1 and CTLA4, might induce T cell responses against tumors. Hence, immune checkpoint inhibitors can restore T cell responses and thus impede the evasion of IR invasive lobular breast cancer cells.

Similarly, activation of CTLA4 on cytotoxic T lymphocytes (CTLs) result in down regulation of the immune system. Antibodies that turn off this inhibitory mechanism allow CTLs to continue to destroy cancer cells.

Preferred therapy targeting PD1, PDL1 and/or CTLA4 comprises antibodies to PD1, PDL1 and/or CTLA4.

Known antibodies that react with PD1 include nivolumab (BMS-936558; Bristol-Myers Squibb, Princeton, NJ), pembrolizumab (MK-3475; lambrolizumab; Merck & Co., Kenilworth, NJ), pidilizumab (CureTech Ltd., Yavne, Israel) and AMP224 and AMP514 (Amplimmune Inc., Gaithersburg, MD).

Known antibodies that react with PDL1 include BMS-936559 (previously MDX-1105; Bristol-Myers Squibb, Princeton, NJ), MSB0010718C (EMD-Serono; Merck KGaA, Darmstadt, Germany), MED14736 (AstraZeneca, London, UK), and MPDL 3280A (Roche, Basel, Switzerland).

Known antibodies that react with CTLA4 include ipilimumab (MDX-010 and MDX-101; Bristol-Myers Squibb, Princeton, NJ), tremelimumab (CP-675,206; Pfizer, New York, NY))

Antibodies to PD1, PDL1 and/or CTLA4 are preferably administered to an individual suffering from an IR invasive lobular tumor by parenteral injection and/or infusion, including intramuscular, intrapleural, intravenous, and subcutaneous injection and/or infusion. A typical treatment schedule or dosing regimen comprises parenteral administration, preferably intramuscular injection, of one dosage unit. The term "one dosage unit", as is used herein, refers to an effective amount of the antibody or antibodies, meaning an amount that produces an effect on the cancer to be treated.

A preferred dosage unit of antibodies to PD1, PDL1 and/or CTLA4 is between 0.1 and 20 mg/kg, preferably between 0.5 and 10 mg/kg. Said dosage unit preferably is applied daily, more preferred every second day, more preferred twice a week, more preferred once a week, more preferred every 2 weeks, more preferred every 3 weeks, more preferred once a month.

Antibodies to PD1, PDL1 and/or CTLA4 are preferably administered together with immune-stimulants. Said immune-stimulants may comprise recombinant, synthetic and natural preparations. Preferred immune-stimulants interleukins such as IL-2, IL-7, and/or IL-12, and interferons, but may also include imiquimod (3-(2-methylpropyl)-3,5,8-triazatricyclo[7.4.0.02,6]trideca-1(9),2(6),4,7,10,12-hexaen-7-amine), synthetic cytosine phosphate-guanosine (CpG), glucans, and cellular membrane fractions from bacteria such as *Mycobacterium* spp., *Corynebacterium parvum, C. granulosum, Bordetella pertussis* and *Neisseria meningitides,* and/or the isolated membrane-bound product N-acetyl muramyl-L-alanyl-D-isoglutamine.

Therapy comprising at least one DNA damaging agent comprises an agent that induces damage to the genomic DNA of a cancerous cell. Said genomic DNA damage includes base modifications, single strand breaks and, preferably, crosslinks, such as intrastrand and interstrand cross-links. A preferred DNA damaging agent is selected from an alkylating agent such as nitrogen mustard, e.g. cyclophosphamide, mechlorethamine or mustine, uramustine and/or uracil mustard, melphalan, chlorambucil, ifosfamide; nitrosourea, including carmustine, lomustine, streptozocin; an alkyl sulfonate such as busulfan, an ethylenime such as N,N'N'-triethylenethiophosphoramide (thiotepa) and analogues thereof, a hydrazine/triazine such as dacarbazine, altretamine, mitozolomide, temozolomide, altretamine, procarbazine, dacarbazine and temozolomide; an intercalating agent such as a platinum-based compound like cisplatin, carboplatin, nedaplatin, oxaliplatin and satraplatin; a topoisomerase I inhibitor such as irinotecan, topotecan, camptothecin and lamellarin D, anthracyclines such as doxorubicin, daunorubicin, epirubicin and idarubicin; mitomycin-C, dactinomycin, bleomycin, adriamycin, mithramycin, and poly ADP ribose polymerase (PARP)-inhibitors such as 3-aminobenzamide, AZD-2281, AG014699, ABT-888, and BMN-673.

A preferred DNA damaging agent comprises bleomycin, cisplatin and/or a topoisomerase 1 inhibitor selected from irinotecan (Pfizer) and topotecan (GlaxoSmithKline), or active metabolites thereof such as SN-38 ((+)-(4S)-4,11-diethyl-4,9-dihydroxy-1H-pyrano[3',4':6,7]-indolizino[1,2-b]quinoline-3,14(4H,12H)-dione), which is the active metabolite of the topoisomerase-I inhibitor irinotecan.

Bleomycin is preferably administered at 0.1-1 USP units/kg, preferably at 0.2-0.5 USP units/kg, whereby USP specifies a potency of 1.5-2 USP units per mg weight. Bleomycin is preferably administered daily, for a period of at least 4 days, more preferred every second day, more preferred twice a week, more preferred once a week. Bleomycin is preferably administered by parenteral injection and/or infusion, including intrapleural, intravenous, intramuscular and subcutaneous injection and/or infusion.

Cisplatin is preferably administered at 10-100 mg/m², preferably at 50-70 mg/m². Cisplatin is preferably administered once a week, more preferred once every two weeks, more preferred once every four weeks. Cisplatin is preferably administered by parenteral injection and/or infusion, preferably intravenous injection.

Irinotecan is preferably administered at 25-500 mg/m², preferably at 100-300 mg/m². SN-38 is preferably administered from about 2 mg/m² to about 150 mg/m², preferably between 50-100 mg/m². Irinotecan and SN-38 are preferably administered once a week, more preferred once every two weeks, more preferred once every three weeks. Irinotecan and SN-38 are preferably administered by parenteral injection and/or infusion, preferably intravenous infusion.

Additional therapy that may be assigned to a patient of which a cancer sample is classified as IR, either alone or in combination with therapy targeting PD1, PDL1 and/or CTLA4 and/or therapy comprising at least one DNA damaging agent, comprises a cyclin dependent kinase (CDK) inhibitor, preferably AT-7519 (4-[(2,6-dichlorobenzene)amido]-N-(piperidin-4-yl)-1H-pyrazole-3-carboxamide; Astex, Cambridge, UK), an inhibitor of nicotinamide phosphoribosyltransferase, preferably FK866 (E)-N-(4-(1-benzoylpiperidin-4-yl)butyl)-3-(pyridin-3-yl)acrylamide; Apoxis, Lausanne, Switzerland) and/or an inhibitor of kinesin spindle protein, preferably SB-715992 (N-(3-Aminopropyl)-N-[(1R)-1-[7-chloro-3,4-dihydro-4-oxo-3-(phenylmethyl)-2-quinazolinyl]-2-methylpropyl]-4-methylbenzamide; Cytolinetics, South San Francisco, USA).

The invention therefore provides anti-Estrogen Receptor directed therapy for use in a method of treating a patient suffering from breast cancer, comprising (a) typing a sample comprising RNA expression products from breast cancer cells from the patient according to a method of the invention, (b) classifying said sample as Immune Related (IR) or Hormone Response (HR), and (c) treating a patient of which the sample is classified as HR with anti-Estrogen Receptor directed therapy.

The invention further provides PD1, PDL1 and/or CTLA4-targeting therapy, and/or a therapy comprising at least one DNA damaging agent, for use in a method of treating a patient suffering from breast cancer, comprising (a) typing a sample comprising RNA expression products from breast cancer cells from the patient according to the method according to the invention; (b) classifying said sample as Immune Related (IR) or Hormone Response (HR); (c) treating a patient of which the sample is classified as IR with therapy targeting PD1, PDL1 and/or CTLA4 and/or with therapy comprising at least one DNA damaging agent.

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following examples, which are provided by way of illustration and not of limitation.

### Figure legends

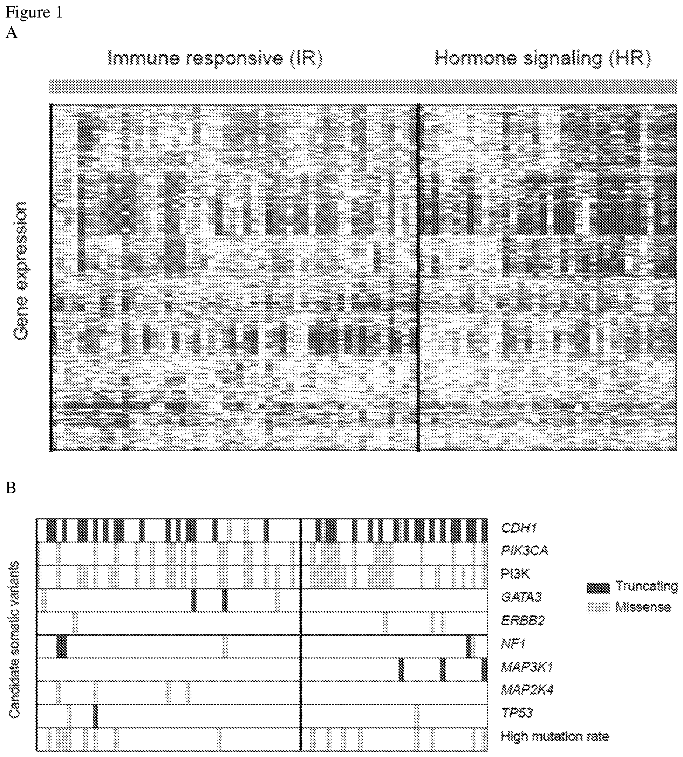

Figure 1 Gene expression clustering reveals two ILC subtypes.
   We defined two robust clusters of ILC samples by consensus clustering on the genome-wide gene expression data: Immune Responsive (IR) and Hormone Receptor signaling (HR). (A) Gene expression of top 250 upregulated and top 250 down-regulated genes in one subtype versus the other. (B) Candidate somatic variants are indicated (truncating mutations in dark and missense mutations in light), while white indicates the absence of variant. PI3K is blue when any of the PI3K pathway genes is mutated. Samples with a high somatic mutation rate (>=10) are indicated at the bottom.
Figure 2 Differential drug response
   The boxplots show the drug response (In IC50) of the cell lines in the IR and HR subtypes for the six drugs with FDR<0.25. The red dotted line is the maximum screening concentration. The first three drugs in the figure are DNA-damaging agents.
Figure 3 Survival analysis of the IR and HR subtypes
   Kaplan-Meier plot of the stratification of the cohort based on the IR and HR subtypes.
Figure 4 Classification performance of ranked genes
   Accuracy of nearest centroid classifier from LOOCV is shown on Y-axis. The number of ranked genes, starting with selected is shown on X-axis.
Figure 5 Classification performance of random subsets of the 300 genes.

### Examples

### Example 1

### Materials and methods

### Study design

All patients with an ILC (based on pathology report) treated in the NKI-AVL since 1980 were extracted from the hospital database. We excluded all patients for which no fresh frozen (FF) tissue was available in the NKI-AVL tissue bank. We selected consecutive tumors without neo-adjuvant treatment and with a preference for those also without adjuvant hormonal therapy. All patients diagnosed with ILC (based on pathology report) treated in the Addenbrookes Hospital Cambridge UK since 1997 and with available FF material were included in this study. Clinical data were extracted from the Addenbrookes Hospital Cambridge database. In some cases, we also sourced FF tissue from adjacent matched normal tissues. Subsequently, we collected matched formalin fixed paraffin embedded (FFPE) tissue blocks for tissue microarray (TMA) construction. The NKI-AVL and Cambridge medical ethical committees approved the study and the use of anonymized archival tissue in this study

### Histopathology

Tumor samples were revised for tumor percentage of the FF material, histological grade and ILC sub-classification (Iorfida et al., 2012. Breast Canc Res Treatment 133: 713-723). TMAs were stained for ER, PR and HER2. Samples were defined to be ER-positive or PR-positive when 10% or more of the tumor cells showed positive staining of ER or PR respectively based on immunohistochemistry. Samples were defined HER2 positive for intensity >=2.

### Survival analysis

We considered only breast cancer specific survival, due to the presence of competing events and (distant) recurrence free survival. For patient stratification in Kaplan-Meier plots, we used Kaplan-Meier estimator and calculated p-values with the logrank test. Cox proportional hazards regression model was stratified by biobank and, unless otherwise specified, fitted with commonly used clinical variables: tumor size, grade, number of positive lymph nodes, treatment (hormonal, radiotherapy and/or adjuvant chemotherapy) and age at diagnosis.

### Microarray Hybridization

Samples with RNA Integrity number (RIN) above 5 (2100 Bioanalyzer, Agilent Technologies) were selected for further analysis. RNA was amplified, labeled and hybridized to the Agendia custom-designed whole genome microarrays (Agilent Technologies) and raw fluorescence intensities were quantified using Feature Extraction software (Agilent Technologies) according to the manufacturer's protocols. Quality of the microarray process was monitored by an internal Agendia quality control (QC) model using >250 control probes related to background issues, general array signal intensity array uniformity and control genes (positive and negative). The microarray expression dataset was imported into R/Bioconductor software (R version 3.02, www.bioconductor.org) for preprocessing (Gentleman et al., 2004. Genome Biol 5: R80).

### Gene expression normalisation

Feature signal intensities were processed and extracted according to the limma Bioconductor R package with background subtraction using an offset of 10. All probe intensities <1 were set as missing values. These missing values were imputed by 10- nearest neighbor imputation (R-package impute) prior to analysis that cannot deal with them. The log2 transformed probe intensities were quantile normalized (Bolstad et al., 2003. Bioinformatics 19: 185-193) using limma. A principal component analysis showed a batch effect for biobank, and an additional batch of samples was identified that were cut at the same time (identifiers RL1110-RL1130). Both batch-effects were adjusted for using ComBat (Johnson et al., 2007. Biostatistics 8: 118-127). Genes with multiple probes were summarized by the first principal component of a correlating subset (all probes with correlation to any other probe >0.5), if such a subset existed or by the most variable probe if no such subset existed. After summarizing by first principal component, signs were adjusted and scaling was carried out to match variance with the most variable probe of gene. Some genes (Wang et al., 2007. Genome Res 17: 1665-1674) showed a discordant signal over multiple probes, so were not summarized and thus kept as separate probes.

### Gene expression clustering

We applied several different clustering algorithms on the top 1000 genes with highest median absolute deviance: hierarchical clustering with Pearson distance and ward D1, single, average and complete linkage, as well as non-negative matrix factorization (NMF). The ward D1, average and NMF methods gave stable clustering results as assessed by consensus clustering. When choosing two clusters, all three methods found largely the same two clusters. To define subtypes, we first performed consensus clustering with average linkage, two clusters, and 90% feature resampling. Then, the consensus matrix was hierarchically clustered with complete linkage and Euclidean distance. Finally, the resulting tree was cut at a quarter of maximum height, defining two big clusters. Samples not falling into one of these two clusters were not assigned to any cluster (n=42). NMF was done with the R package NMF, consensus clustering with the ConsensusClusterPlus package. To assign cell lines to clusters, we normalized together the raw gene expression data of cell lines and tumor samples. Then, we applied the same clustering approach described above, but cut the tree at maximum height such that all cell lines where assigned to a cluster. All tumor samples assigned to a cluster were assigned to the same cluster in both clustering results with and without cell lines.

### Gene optimization

To determine the genes required for classification of ILC tumors in one of the two subtypes, we obtained a measure of feature importance from a random forest classifier (Breiman, 2001. Machine Learning 45: 5-32). We used 10 000 a bootstrapped trees with 100 randomly selected features each. Feature importance was measured by the mean decrease in Gini index upon resampling of that feature. Differential expression between the two subtypes was tested with a Mann-Whitney U test, p-values were corrected according to the Benjamini-Hochberg method for controlling the false discovery rate (FDR).

### Validation dataset

We used the ILC samples of METABRIC (Curtis et al., 2012. Nature 486: 346-352) as a validation set for the gene expression subtypes. 76 samples are in common between RATHER and METABRIC, and were removed from the validation set, resulting in a set of 103 samples. We mapped probes to genes with the ReMOAT annotation (Barbosa-Morais et al., 2010. Nucl Acids Res 38: e1741).

### Drug sensitivity

Drug sensitivity was assessed on the Sanger cell line panel (internal version 17). We used the cell lines common in our ILC cell line panel and in the Sanger cell line panel. We used cell line AU565 instead of SK-BR-3, which is derived from the same patient. Among the 262 drugs, we focused our assessment on 88 agents that had measurement in at least three cell lines per subtype. With this dataset, we performed a two-sided t-test between the AUC of the dose-response curves of the cell lines in the two subtypes, correcting for multiple testing with the Benjamini-Hochberg method.

### Results

### Genomic profiling of ILCs

To explore the biology of invasive lobular carcinomas (ILCs), we performed comprehensive molecular profiling of 144 untreated tissue samples from primary ILC tumors with 6.8 years median clinical follow-up.

We first used gene expression data to explore the biological processes at play in ILC. Extensive clustering stability analysis based on a variety of clustering approaches identified two robust expression subtypes (Figure 1A). Based on a gene sub-sampling analysis, we found that we could stably classify 71% (102/144) of samples into one of two subtypes (63 and 39 samples). Among these 102 samples, 89 were characterized by mutation analysis and gene expression data and are represented in Figure 1.

A first subtype, referred to as immune responsive (IR), shows upregulation of genes characteristic of cytokine/chemokine signaling and normal-like breast cancer. GATA3 mutations were found to be enriched in the IR subtype, even though not significantly so (Figure 1B). The IR subtype shows up-regulation of a range of lymphoid signaling molecules at the mRNA level, such as TGFBR2, IL11RA, TNFRSF17, CCL15, CCL14, CCR2, CD27, XCL2, IFNAR2, CD40LG, suggesting alterations in either the make-up or functional activity of immune cells within these tumors. Interestingly, negative regulators of the immune response PDCD1 (PD1), CD274 (PDL1) and CTLA4 are expressed at higher mRNA levels in the IR subtype (Data not shown). Further expression analyses suggested that the immune infiltrate in the IR subtype is enriched in T cells.

A second subtype, referred to as hormone receptor (HR) signaling subtype, shows up-regulation of estrogen receptor (ER) and progesterone receptor (PR). The HR subtype is characterized by ERBB2 mutations (Figure 1B). Collectively, these findings support elevated levels of hormone receptor signaling in this subtype.

### The two ILC subtypes are validated in an independent dataset

To validate the existence of hormone receptor signaling and immune responsive ILC subtypes, we investigated the ILC samples of the METABRIC consortium (Curtis et al., 2012. Nature 486: 346-352)]. Using the same clustering approach de novo on the METABRIC gene expression data, we also identified two robust subtypes (data not shown).

### Modeling therapeutic response in cell line models

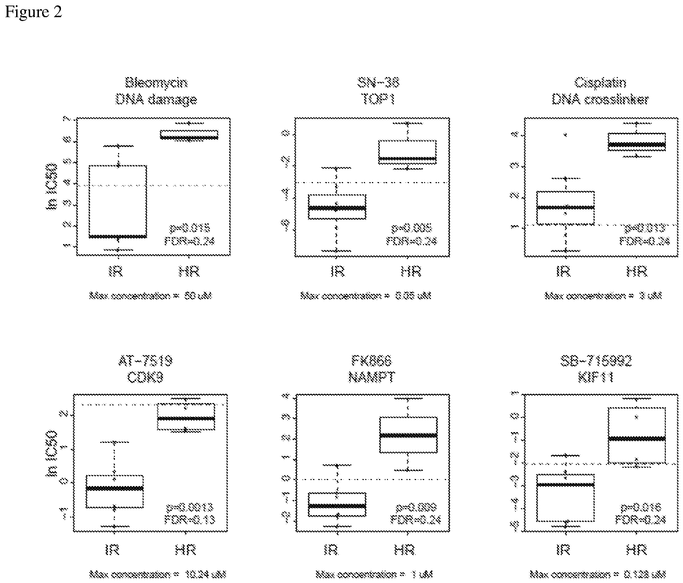

To identify candidate therapeutic options, we profiled a set of 15 ILC cell lines as in *vitro* models. We used their gene expression profiles to map them to the IR (10 cell lines) and HR (5 cell lines). We then used the response data for 88 drugs on a subset of these cell lines, as provided by the Wellcome Trust Sanger Institute (Garnett et al., 2012. Nature 483: 570-575), to test for differential drug sensitivity between the subtypes. We retained six drugs at an FDR<0.25 (Figure 2). We found that cell lines of the IR subtype are more sensitive to three different DNA-damaging agents: Bleomycin, Cisplatin and the topoisomerase 1 inhibitor SN-38. This suggests that the IR subtype may be more responsive to chemotherapy. Conversely, we did not find any drugs showing a significantly larger effect in the HR subtype.

### Survival analysis

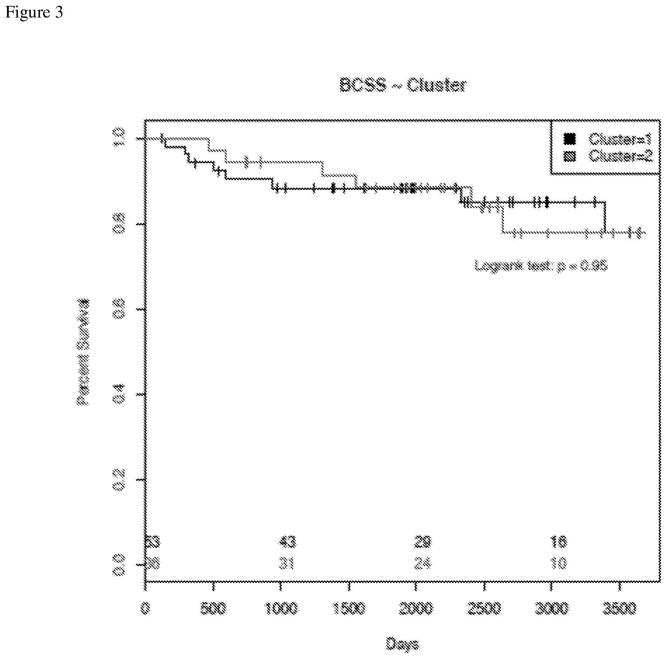

Kaplan-Meier plot of the stratification of the cohort based on the IR and HR subtypes are shown in Figure 3. There is no significant difference in survival.

### Gene optimization

A total of 300 genes were identified that were differentially expressed between IR and HR ILCs (see Table 1). In this Table, +1 indicates that the gen is upregulated in IR, while -1 indicates that the gene is downregulated in IR, when compared to a level of expression in HR, or an average level of expression in ILCs. To assess classification performance of a set of genes we trained a nearest centroid classifier. Classification performance was measured by accuracy in leave-one-out cross validation (LOOCV).

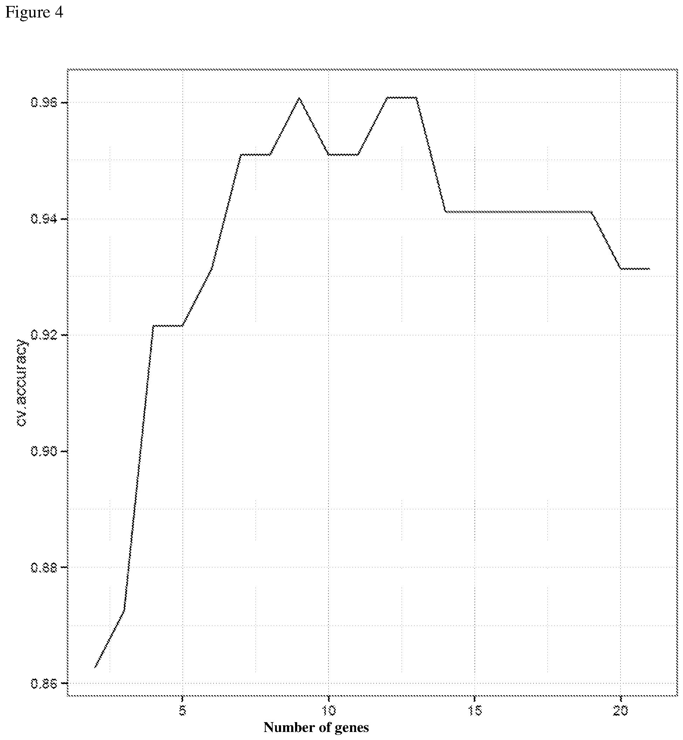

As can be seen in Figure 4, maximum classification performance is reached with the top 9 ranked genes, while the top 2 ranked genes (GIMAP6 and P2RY14) already show good performance.

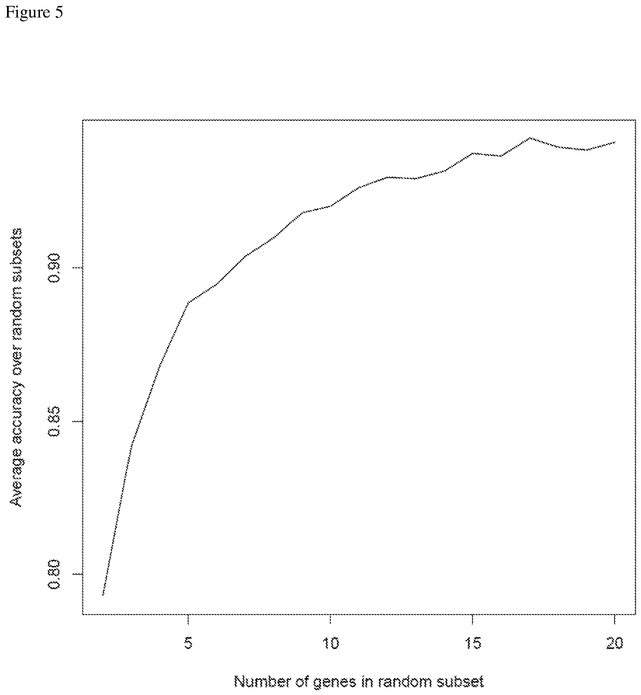

Using 100 random subsets for each number of genes indicated on the X-axis, it is evident that 2 randomly selected genes already allow discrimination between the two subsets (Figure 5). A good performance is obtained with 5 randomly selected genes from the group of genes indicated in Table 1. Performance keeps increasing up to 18 randomly selected genes.

**Table 1**

| **Seq ID No** | **Gene** | **Sequence** | **Mean Decrease Gini** | **Sign** |
|---|---|---|---|---|
| 1 | GIMAP6 | CCATCTTCCCTAGACACAGCAGACATCTGAGAAAGCTTCAGCATTTCTCTTGCTAACAGA | 0.3728383 | 1 |
| 2 | P2RY14 | TTTTTCTGGAAAACAGACGGATTTTACTTCTGGAGACATGGCATACGGTTACTGACTTAT | 0.3140863 | 1 |
| 3 | CD79B | CGCTGAAGGATGGTATCATCATGATCCAGACGCTGCTGATCATCCTCTTCATCATCGTGC | 0.3070698 | 1 |
| 4 | GIMAP5 | TCATTGTTCTAATAATCACCAATTCAGACTCAGATCCTCGTGGTCTATGGAGCATGCTGC | 0.2810331 | 1 |
| 5 | GIMAP8 | TTCTTCTGGGTAGATACAGGATAAGATAGATACAGGGTAAAATGTTGAGCACTTTGTACA | 0.2735827 | 1 |
| 6 | LOC100290115 | CTTCAAATGAACAGCCTGAGAGCCGAGGACACGGCCGTGTATTACTGTGCGAGAGACACA | 0.2561993 | 1 |
| 7 | IL33 | AACTTCATTTCTGTATCACCTGACCTCTGGATGCCAAAACGTTTATTCTGCTTTGTCTGT | 0.2527823 | 1 |
| 8 | SEL1L3 | CTGCTAAGGTAGTGAATAAATCAGTAATGCAATATTGTGGGTCCAAACTACTCTTTGCAC | 0.2440941 | 1 |
| 9 | SLC9A9 | TTTTTTGCTTTCCAAACAGAATCTCTGGGGCACAAGTTTTACACTCAAGCTAAGTATAAC | 0.2420411 | 1 |
| 10 | CLIC2 | CACCTCATGGTCAGAGATGAGAATGTAGCCTTTGTGACCAGATTATATTATTTTTAAATG | 0.2402659 | 1 |
| 11 | ICAM2 | CTGTGACAGGCAGCAGAGACTTGGGACATTGCCTTTTCTAGCCCGAATACAAACACCTGG | 0.2387606 | 1 |
| 12 | IL11RA | GAGCCCATTTCTGTGAGACCCTGTATTTCAAATTTGCAGCTGAAAGGTGCTTGTACCTCT | 0.233638 | 1 |
| 13 | GIMAP7 | TTTGGGAAGTCAGCCATGAAGCACATGGTCATCTTGTTCACTCGCAAAGAAGAGTTGGAG | 0.2309312 | 1 |
| 14 | LOC652159 | AAATGAACAGCCTGAAAACCGAGGACACGGCCGTGTATTACTGTGCTAGAGACACAGCGA | 0.2296262 | 1 |
| 15 | MEI1 | AGAATGAGACCTGGAGACAAAGGGCATAATTGTTGGGGAAATGGATGACAGCTGAAGCTA | 0.2174771 | 1 |
| 16 | MAL | GCCCCATCTTGTGCCATGTTTTAAGTCTTCATGGATGTTCTGCATGTCATGGGGACTAAA | 0.2112442 | 1 |
| 17 | LOC652106 | CTCCAGGGAAGGGGCTGGAGTGGGTTTCATACATTAGTAGTAGTAGTAGTACCATATACT | 0.2097388 | 1 |
| 18 | LOC642838 | CAGCAGCCTGCAGCCTGAAGATTTTGCAGCTTATTACTGTCAACAGAGTGACAGTACCCC | 0.2083823 | 1 |
| 19 | POU2AF1 | TTTTCTGGGAAATGACTTTTCTGGGAAATGACAGTTTCTTTGACATATTTTCTTTGCCCA | 0.2071212 | 1 |
| 20 | IGHA1 | TGCTGAGTTGGGTTTTCCTTGCTGCTATTTTAAAAGGTGTCCAGTGTGAGGTGCAGCTGG | 0.2065839 | 1 |
| 21 | LOC100132941 | ATTAAAAGGTGTCCAGTGTGAGGTTCAGCTGGTGCAGTCTGGGGGAGGCTTGGTACATCC | 0.2062982 | 1 |
| 22 | AMPD1 | GGAATTTCTCATGAGGAGAAAGTAAAGTTTCTGGGCGACAATTACCTTGAGGAAGGCCCT | 0.2032299 | 1 |
| 23 | MZB1 | GGACATGTTTGCACTACTTGGGGGAGTTTGGAGAAGACCAGATCTATGAAGCCCACCAAC | 0.2031787 | 1 |
| 24 | TGFBR2 | CATGCTGATACCATCCCAATAGCTGTTGCCCATTGACCTCTAGTGGTGAGTTTCTAGAAT | 0.2019837 | 1 |
| 25 | ENST00000398668 | GCACAAGCTACGCAGACTCCATGAAGGGCCAATTCACCATCTCCAGAGACAATGCTAAGA | 0.1992365 | 1 |
| 26 | IGKV1.27 | TGATCTATGCTGCATCCAGTTTGCAGTCGGGGGTCCCATCTCGGTTCAGTGGCAGTGGAT | 0.1961856 | 1 |
| 27 | FAM107A | TCTCCTTTGCAGCTTAGGATGGCTATGTGATCAGGTGTGGCCAATGAAATTGAAGAGGAA | 0.1925129 | 1 |
| 28 | SPNS2 | GCTGCTGATTGTGAATCTCAGAGTCTTAAGAGAGAAGCCAAATATATTCCTCTTGTAAAT | 0.191244 | 1 |
| 29 | LOC100290819 | GGCAGCGGATATGGAAGAGATTTCACTCTCACTGTCAGCAGCCTGCAGCCTGAAGATTTT | 0.190084 | 1 |
| 30 | GIMAP1 | ACGGAAGGAATCCTGTAGTTTCAGGCATAGTTTTAATGACACAGAAAACTTTGCTGCATC | 0.1861181 | 1 |
| 31 | FLI1 | GGGCTGTTTAAGTCACTGACTTATGAGAAAGCAAAGCACTGGTACAGTTATTTAACAGGC | 0.185451 | 1 |
| 32 | B4GALNT4 | TACAAGTCGGACTTTGACCGGGTTGGAGGAATGAACACGGAGGAGTTCCGAGACCAGTGG | 0.1843703 | -1 |
| 33 | LOC100290415 | ACACAGTCTACATGGAGCTGAGAAGCCTGAGATCTGAGGACACGGCCGTGTATTACTGTG | 0.1814927 | 1 |
| 34 | KIAA0125 | CCATTTTAAAGATGGCTACTTAGGACCATATGGATGTTGTACTGATGTCATTTGACCACG | 0.1789083 | 1 |
| 35 | IGKV1.16 | GACAGAGTCACCATCACTTGTCGGGCGAGTCAGGGAATTAGCAATTATTTAGCCTGGTTT | 0.1762107 | 1 |
| 36 | IGJ | TTGGGTGATGTAAAACCAACTCCCTGCCACCAAAATAATTAAAATAGTCACATTGTTATC | 0.1736023 | 1 |
| 37 | LRMP | AGGTTCTCAGAATGACCGTAAGATAGCTTACATTTCCTCTTTTTGCCTTTATCTCCCCAA | 0.1721281 | 1 |
| 38 | IGKV1D.33 | GCAGCCTGCAGCCTGAAGATATTGCAACATATTACTGTCAACAGTATGATAATCTCCCTC | 0.169694 | 1 |
| 39 | SVEP1 | TCTCGCTGTCATACAGCTGTTTGCCAGTCTCCCTGCTTAAATGGTGGAAAATGTGTAAGA | 0.1695933 | 1 |
| 40 | IRF4 | TTTTGTATTGATTTTCACAGCTTTGAGGAACATGCATAAGAAATGTAGCTGAAGTAGAGG | 0.1685261 | 1 |
| 41 | IGKV3.7 | AGCAGCCTGCAGCCTGAAGATTTTGCAGTTTATTACTGTCAGCAGGATTATAACTTACCT | 0.1651686 | 1 |
| 42 | LOC100287723 | GCAGATTACACTCTCACCATCCGCAGCCTGCAGCCTGAAGATTTTGCAAATTATTACTGT | 0.1640216 | 1 |
| 43 | IGL | CCCAAGGCATCAAGCCCTTCTCCCGTGCACTCAATAAACCCTCAATAAATATTCTCATTT | 0.1629227 | 1 |
| 44 | IGKC | CCATCAGCAGCCTGCAGTCTGAAGATTTTGCAGTTTATTACTGTCAGCAGTATAATAACT | 0.1622682 | 1 |
| 45 | CASP10 | CCCTAAGTTTCCTGGCATTTCTAGAGAAACAAGGTAAAATAGATGAAGATAATCTGACAT | 0.1585399 | 1 |
| 46 | IGKV1D.27 | TAGGAGACAGAGTCACCATCACTTGCCGGGCAAGTCAGACCATTAGCAGCTATTTAAATT | 0.1575712 | 1 |
| 47 | IGKV4.1 | GGCCACCATCAACTGCAAGTCCAGCCAGAGTGTTTTATACAGCTCCAACAATAAGAACTA | 0.1569229 | 1 |
| 48 | ITM2A | CTAGTTGCTGTGGAGGAAATTCGTGATGTTAGTAACCTTGGCATCTTTATTTACCAACTT | 0.1565255 | 1 |
| 49 | LOC100133862 | CAGGCCCCTGGACAAGGGCTTGAGTGGATGGGAAGGATCATCCCTATCCTTGGTATAGCA | 0.1551818 | 1 |
| 50 | TTLL12 | GAGGGCAGGGCTAGGTGTAGCGGTGTCTCCTCTTTGAAATTAAGAACTATCTTTCTTGTA | 0.1541169 | -1 |
| 51 | LOC100289290 | GTGGCACATACTATGCAGACTCCGTGAAGGGCCGATTCACCATCTCCAGAGACAATGCCA | 0.1538251 | 1 |
| 52 | IGHV1.46 | ACAGAAGTTCCAGGGCAGAGTCACCATGACCAGGGACACGTCCACGAGCACAGTCTACAT | 0.1508526 | 1 |
| 53 | PTGFR | CCACCTGGTAAATAATGTTGGACTTCAACTTGTTACTGGCACATTTTAATAGTAGATGAA | 0.1494197 | 1 |
| 54 | C10orf54 | AGATCTGTCAACAGGTTAAGTCAATCTGGGGCTTCCACTGCCTGCATTCCAGTCCCCAGA | 0.143229 | 1 |
| 55 | MGC16025 | GCCTCTGACCCTGATGAAATAGCTTCGGTGGCATTTGCATCAAGATCATGTTAGTGTCAT | 0.1420385 | 1 |
| 56 | PECAM1 | TTCAAAGGCTTGTAGTTTTGGCTAGTCCTTGTTCTTTGGAAATACACAGTGCTGACCAGA | 0.1413514 | 1 |
| 57 | PIP5K1B | GACATACGCTCCATTAGCATTCCGATATTTCAGAGAACTTTTTGGTATCAAGCCTGATGA | 0.1412967 | 1 |
| 58 | BMX | TTATGCTGCTCCTGATATAACACTTTCCAGCCTATAGCAGAAGCACATTTTCAGACTGCA | 0.1409081 | 1 |
| 59 | ENST00000354689 | CTGGGTTCGCCAGGCTCCAGGGAAGGGACTGGAGTGGGTTTCATACATTAGTGGTAATAG | 0.1385273 | 1 |
| 60 | CLIC5 | TAAAATACGTTGCCTCAATATAAGGTTTGGGCTATTCTGTGTTTCTATAGAAGCAATCTG | 0.138488 | 1 |
| 61 | MEOX2 | GGACATATTCTTGTACCACAGACAAAACAAATCTTATGTTGCATTTACTATCAACTGCTG | 0.1376656 | 1 |
| 62 | SOD3 | AGAGCTGCTCCTTTGGGGGAATGTTTGGCAACCTTTGTGTTACAGATTAAAAATTCAGCA | 0.1371701 | 1 |
| 63 | AY339598 | GTCTGAAGATTTTGCAACTTATTACTGTCAACAGTATTATAGTTTCCCTCCGACGTTCGG | 0.1355871 | 1 |
| 64 | CTA.246H3.1 | AACAAGGCCACACTGGTGTGTCTCATGAATGACTTCTATCTGGGAATCTTGACGGTGACC | 0.1333871 | 1 |
| 65 | DNASE1L3 | GACCCAAGGGTCTCATCTTATTAACCATTTCTTGCCTCTAAATAAAATGTCTCTAACAGA | 0.1317709 | 1 |
| 66 | AV736391 | TCGCAGCTACCTGAGTCAGACCTGTGCTTTTTCACCTCTACGGAAGATGTCAGAGCGTTT | 0.1313191 | 1 |
| 67 | IGHV3.23 | AGTTTGGGCTGAGCTGCCTTTTTCTTGTGGCTATTTTAAAAGGTGTCCAGTGTGAGGTGC | 0.1310658 | 1 |
| 68 | IGHV4.59 | GTGCAGCTGCAGGAGTCGGGCCCAGGACTGGTGAAGCCTCCGGGGACCCTGTCCCTCACC | 0.1302513 | 1 |
| 69 | IGHV3.33 | ATGGTATGATGGAAGTAATAAATACTATGCAGACTCCGTGAAGGGCCGATTCACCATCTC | 0.1279853 | 1 |
| 70 | METTL7A | TATGGAAGCTGTGAAAATCATCACAAGTGCCTCTGAAAGCGAGTGTTAGGTTGGTTAGAG | 0.1276967 | 1 |
| 71 | LOC390712 | CTGTGAAGGGCAGATTCACCATCTCCACAGACAACTCAAAGAACACGCTCTACCTGCAAA | 0.1273474 | 1 |
| 72 | CD38 | TGAAAAATCCTGAGGATTCATCTTGCACATCTGAGATCTGAGCCAGTCGCTGTGGTTGTT | 0.1242059 | 1 |
| 73 | ENST00000443661 | TGCAATCTGGGTCTGAGTTGAAGAAGCCTGGGGCCTCAGTGAAGGTCTCCTGCAAGGCTT | 0.1213082 | 1 |
| 74 | ANXA2R | GCAGAAGGCTTTTTGGATTCTCCTCCTTGAAAAGATTCTCAGTTACCAAACGTCTCCACC | 0.121285 | 1 |
| 75 | LPIN1 | ATTACACTTTCAAAGAGAATTCCCTTTGCAATTTTATGTTTGGATCACCACTGTAAGCAC | 0.1210672 | 1 |
| 76 | ENST00000411420 | CTGAGACTGTCCTGTCCAGCCTCTGGATTCACCTTCAGTAACCACTACATGAGCTGGGTC | 0.1205925 | 1 |
| 77 | RYR1 | GAGTCTTATGTCTGGAAGATGTACCAAGAGAGATGTTGGGATTTCTTCCCAGCTGGTGAT | 0.120091 | 1 |
| 78 | CLEC1A | GCACCTGTAATAGTTTCAGTTCCTATTTTCTTCCATTGACCCATATTTATACCTTTCAGG | 0.118806 | 1 |
| 79 | INMT | GAGAGAGCTAGTGTAAGCTGTTGATGAGAGCTGTTGCTGAATAAAACCATATTCACCTGC | 0.1176619 | 1 |
| 80 | ACAP1 | GTTCACTTCTCCAAAGTCCGGTCTCTGACCCTTGACTCATGGGAGCCAGAACTAGTGAAG | 0.1175861 | 1 |
| 81 | KLRB1 | TCAACCCTTGGAATAACAGTCTAGCTGATTGTTCCACCAAAGAATCCAGCCTGCTGCTTA | 0.1171576 | 1 |
| 82 | TANC2 | AGGTTTTCTATGACTCAGATGTAAAGGACTTTCTCTGTACAGTATATTATCCAATGCATG | 0.1169085 | |
| 83 | PTGDS | CAAAGCAACCCTGCCCACTCAGGCTTCATCCTGCACAATAAACTCCGGAAGCAAGTCAGT | 0.1159179 | 1 |
| 84 | IGLV2.14 | CATCACTGGTCTCCAGGCTGAGGACGAGGCTGATTATTACTGCAGCTCATATACAAGCAG | 0.1159175 | 1 |
| 85 | IGHV3.66 | TCTCAGTTATTTATAGCTGTGGTAGCACATACTACGCAGACTCCGTGAAGGGCCGATTCA | 0.1138371 | 1 |
| 86 | TFPI | GCCTTCTGCATTCATGCATCCATGTTCTTTCTAGGATTGGATAGCATTTCATGCCTATGT | 0.1130304 | 1 |
| 87 | FCRL1 | CAAGAGTTCACCTACCTCAACTCACCTACCCCAGGGCAGCTACAGCCTATATATGAAAAT | 0.112808 | 1 |
| 88 | PGM5 | CTAACAGCCAGCCACTGCCCTGGAGGACCAGGGGGAGAGTTTGGAGTGAAGTTTAATGTT | 0.1110997 | 1 |
| 89 | IGLL1 | TCCAAGCCAACAAGGCTACACTGGTGTGTCTCATGAATGACTTTTATCCGGGAATCTTGA | 0.1104732 | 1 |
| 90 | DARC | TCCCCCTCAACTGAGAACTCAAGTCAGCTGGACTTCGAAGATGTATGGAATTCTTCCTAT | 0.109899 | 1 |
| 91 | CCL14 | ACTATATCAAGGACATGAAGGAGAACTGAGTGACCCAGAAGGGGTGGCGAAGGCACAGCT | 0.1085745 | 1 |
| 92 | LOC400456 | TTACATCAGTATCATCATTCCAAGAAGCGGTATCCAAGCTGTTTTATCGAAAGTGCGCAT | 0.1075516 | 1 |
| 93 | C7 | ACAGGCTGGCTGCGTGTTCTTGAAATAGGTGTTACCTTCTCTGGGCCTTGGTTTTTTAAA | 0.1047268 | 1 |
| 94 | CR616309 | GCCCCATTTCAAGTATAACCAGGAGGGAAAATGGTGCTTGAAATAAGCATGCCACAAAGG | 0.1019357 | 1 |
| 95 | NKAIN1 | CCAGGTTCTCTTTTCACTGGGACCTTGCAAGGAGGGGAGTGCTCTCCTGGTTTCTGTGCA | 0.1010148 | -1 |
| 96 | TNXB | AGTTCTCGGTGCCCTTCACGGAAATGAAGCTGAGACCAAGAAACTTTCGCTCCCCAGCGG | 0.1000135 | 1 |
| 97 | MMP11 | GGCCAAAAAGTTCACAGTCAAATGGGGAGGGGTATTCTTCATGCAGGAGACCCCAGGCCC | 0.0999101 | -1 |
| 98 | IGLV6.57 | AACTCTGCCTCCCTCACCATCTCTGGACTGAGGACTGAGGACGAGGCTGACTACTACTGT | 0.0990432 | 1 |
| 99 | SHE | TGCCCTTTCCTGCGGAAAGGGTGTTTTGAAGTCTAATACAACTATCATCACAAGGTCCCT | 0.0970628 | 1 |
| 100 | IGHV3.73 | CCAGAGATGATTCCAAGAACACGGCGTATCTGCAAATGAACAGCCTGAAAACCGAGGACA | 0.0941202 | 1 |
| 101 | ZNF366 | CCACCGGCCATGAGACTAAGGCTGACCTTTCATAAACCCACAAGAGTAACTGGTATATTT | 0.0935525 | 1 |
| 102 | MALL | CTTGAAAAGCTTCTATGTGTCTCTCCTTTTGTTGCCTGGCAGCTGTCTAGGATGATCACT | 0.0930558 | 1 |
| 103 | PLCG2 | GAGTCAGCAACAGCAAGTTTTACTCATAGAAGCTGGGGTATGTGTGTAAGGGTATTGTGT | 0.0929387 | 1 |
| 104 | IGHV1.2 | CCTCAGTGAAGGTCTCCTGCAAGGCTTCTGGATACACCTTCACCGGCTACTATATGCACT | 0.0924283 | 1 |
| 105 | PPP2R2B | AGTTTTCTCTGGTGGGTCCAGTGTTTTGTTCCTAGGTGTCTGCTGCGATAAAATGAGGTT | 0.0920305 | 1 |
| 106 | IGHV3OR16.7 | CCGTATCTGCAAATGAACAGCTTGAGAGCTGAGGACACGGCTGTGTATTACTGTGTGAAA | 0.0915109 | 1 |
| 107 | IGKV2D.29 | TTGTATTGGTACCTGCAGAAGCCAGGCCAGCCTCCACAGCTCCTGATCTATGAAGTTTCC | 0.0909582 | 1 |
| 108 | IGKV2.18 | GGGCAGTCTCCACAGCTCCTGATTTATAGGGTTTCCAATCATCTTTCTGGGGTCCCAGAC | 0.0895279 | 1 |
| 109 | BC144530 | TATTTGAATTGGTACCTGCAGAAGCCAGGCCAGTCTCCACAGCTCCTGATCTATGAGGTT | 0.0892731 | 1 |
| 110 | ACSL5 | CTGCTCTACCTTACCCACAGATAACACATGTTGTTTCTACTTGTAAATGTAAAGTCTTTA | 0.088697 | 1 |
| 111 | TXNDC5 | GGCAGAGACCTTGACTCGTTACACCGCTTTGTCCTGAGCCAAGCGAAAGACGAACTTTAG | 0.0879397 | 1 |
| 112 | BC045756 | TGGGTGTTTTTTGGTTTTTGGTTTCTGGTTTACAATCTCGTCATTCAACAAAGATGGGAG | 0.0873954 | 1 |
| 113 | C10orf105 | CCAGAGACACCAGATCTTCTGATTTCTCATGAGAAATTGAACATCTGAGCTTTTAAATAA | 0.0871425 | 1 |
| 114 | ZDHHC18 | AGATATTGTTCCACCATCCCCCTCCTTGGCCCTTCAAGTGGGCTGAAGCCCTTGGAAAGT | 0.086863 | 1 |
| 115 | FAM180B | GGTGGGCTGGAGCAGGTGTGGCCCTGATTCTGTGTTGCTCTTCTCATAAAATGTTCTGTT | 0.0867138 | 1 |
| 116 | VAMP5 | CTGCAGCAGCGTTCAGACCAACTCCTGGATATGAGCTCAACCTTCAACAAGACTACACAG | 0.0860009 | 1 |
| 117 | CPNE5 | TGGTTCTGTGCCCGTCTCTGAGACAGTCTCTGTGTGGAATTTGCCTTAAACTGAAGTAAA | 0.0855679 | 1 |
| 118 | IGHG1 | GCGTGACCATCTCCTGGACCCGCCAGAATGGCCAAGCTGTGAAAACCCACACCAACATCT | 0.0833098 | 1 |
| 119 | TARP | CTGGACAAAGAACACAGATGTATCGTCAGACATGAGAATAATAAAAACGGAGTTGATCAA | 0.0823656 | 1 |
| 120 | MUSTN1 | AGACTGTCTTTGAGAAGCCCAAAGCCGGACCCACCAAGAGTGTCTTCGGCTGAGAAGTGT | 0.0797104 | 1 |
| 121 | TNFRSF17 | GATCTCTTTAGGATGACTGTATTTTTCAGTTGCCGATACAGCTTTTTGTCCTCTAACTGT | 0.0791632 | 1 |
| 122 | CFHR3 | TCAGGAAGTTACTGGGATTACATTCATTGCACACAAAATGGGTGGTCACCAGCAGTACCA | 0.0788775 | 1 |
| 123 | BIN1 | AGCAAAGGGAAATCAAGAGGAGACCCCCAGGCAGAGGGGCGTTCTCCCAAAGATTAGGTC | 0.0781509 | 1 |
| 124 | WHSC1 | TCCTACTGCTGTGAGCATGACTTAGGGGCGGCATCGGTCAGAAGCACCAAGACTGAGAAG | 0.0772003 | -1 |
| 125 | IGKV1D.13 | GAAAGCTCCTAAGCTCCTGATCTATGATGCCTCCAGTTTGGAAAGTGGGGTCCCATCAAG | 0.0764297 | 1 |
| 126 | SLAMF1 | TCTGTGCCTCAGTTTCTCTCTCAGGATAAAGAGTGAATAGAGGCCGAAGGGTGAATTTCT | 0.0753424 | 1 |
| 127 | ADRB2 | CTCTTATTTGCTCACACGGGGTATTTTAGGCAGGGATTTGAGGAGCAGCTTCAGTTGTTT | 0.0749854 | 1 |
| 128 | LOC642891 | AGCGAAGGCAACTCATGATTCTGCCTCAGAAGCAATCGAATAAAATATTTTGAAAATGAA | 0.0746785 | 1 |
| 129 | FBLN5 | GGGAACCCTGGGAGTAGCTAGTTTGCTTTTTGCGTACACAGAGAAGGCTATGTAAACAAA | 0.0745848 | 1 |
| 130 | PNOC | GCCACTGCCATAACTTGTTTGTAAAAGAGCTGTTCTTTTTGACTGATTGTTTTAAACAAC | 0.0744541 | 1 |
| 131 | IGKV2D.28 | GATTTTACACTGAAAATCAGCAGAGTGGAGGCTGAGGATGTTGGGGTTTATTACTGCATG | 0.0739373 | 1 |
| 132 | IGKV2D.24 | TGCTAATGCTCTGGGTCCCTGGATCCAGTGGTGATATTGTGATCGCCCAGACTCCACTCT | 0.0737708 | 1 |
| 133 | IG KV6D.41 | TTACCATCAGTAGCCTGGAAGCTGAAGATGCTGCAACATATTACTGTCAGCAGGGCAATA | 0.0734206 | 1 |
| 134 | LDLRAD2 | GTGGTAGCTACTGAGATCACCAGATCTTGTTATTTGATGTGTTAATAAAGAAGCACATAT | 0.0724355 | 1 |
| 135 | ABCB1 | GTCACTGCCTAATAAATATAGCACTAAAGTAGGAGACAAAGGAACTCAGCTCTCTGGTGG | 0.0718928 | 1 |
| 136 | BMP8A | TAGAGGTGCTGCTGTATTTACCTGAGAGCTATGCTTTTCATCGAAAACCTAAACGTGATC | 0.0712076 | -1 |
| 137 | LOC100293277 | GGGATGAGGCCGACTATTACTGTCAGGTGTGGGATAGTAGTAGTGATCATCATCCCACGGTGA | 0.0704335 | 1 |
| 138 | X72475 | GCCTGAAGATATTGCAACATATTACTGTCAACAGTATGGTAATCTCCCATTCACTTTCGG | 0.0701583 | 1 |
| 139 | IGHV3.49 | ACAACAGAATACGCCGCGTCTGTGAAAGGCAGATTCACCATCTCAAGAGATGATTCCAAA | 0.0698637 | 1 |
| 140 | CASP5 | ACCGCACAAGGGGCTCCATCTTCATTACGGAACTCATCACATGCTTCCAGAAATATTCTT | 0.0697763 | 1 |
| 141 | IGLV1.47 | ATACTGGTACCAGCAGCTCCCAGGAACGGCCCCCAAACTCCTCATCGAAAGGAATAATCA | 0.0695112 | 1 |
| 142 | HLA.DOB | CCAAATCATTTACTTTTCTGTGGTCCAGCCCTACTCCTATAAGTCATGATCTCCAAAGCT | 0.0692694 | 1 |
| 143 | PDE2A | ACCTGCCTTACCTTTCTGAGTTGCCTTTAGAGAGATGCGTTTTTCTAGGACTCTGTGCAA | 0.0684447 | 1 |
| 144 | KLRG1 | GTGTCCCTTTGCAGATCAAGCTTTATTCTGAAGAATAAACCTAGCTGGCATGCTGGTGTG | 0.0683712 | 1 |
| 145 | IGLV3.1 | ATTCTCTGGCTCCAACTTTGGGAACACAGCCACTCTGACCATCAGCGGGACCCAGGCTAT | 0.0681577 | 1 |
| 146 | C16orf54 | GTATCACTTCTGTAAATGGAAAACCAGTCTCATTTGCCATCAATAGAAGGTAAAACATGA | 0.0667206 | 1 |
| 147 | LOC96610 | ATTTATGACAATAATAAGCGACCCTCAGGGATTCCTGACCGATTCTCTGGCTCCAAGTCT | 0.0665083 | 1 |
| 148 | PIM2 | GGTGAGGGGACCCTACTTTGTTATCCCAAGTGCTCTTATTCTGGTGAGAAGAACCTTAAT | 0.065868 | 1 |
| 149 | SELP | AAATACCTCTTTATTTTTTGATTGAAGGAAGGTTTTCTCCACTTTGTTGGAAAGCAGGTG | 0.065686 | 1 |
| 150 | PTGER4 | CCCAAACGTGGTTACATTAGCCATTCATGTATGTCAGAAGTGCAGAATTGGGGCACTTAA | 0.0653338 | 1 |
| 151 | SLAMF7 | GGAGACCTCCCTACCAAGTGATGAAAGTGTTGAAAAACTTAATAACAAATGCTTGTTGGG | 0.0648325 | 1 |
| 152 | ABCA8 | TGATGGTTTATAAGTTGCCAGTGGAAGATGTGCAACCTTTAGCCCAAGCTTTCTTCAAAT | 0.064788 | 1 |
| 153 | DDX26B | TGCAAGGACCTCTGGAGATGAAGAAACAGTTTGTTGAATTTACCATCAAGGAAGCCGCAA | 0.0642466 | 1 |
| 154 | COL10A1 | ATACAGATTTGAGCTATCAGACCAACAAACCTTCCCCCTGAAAAGTGAGCAGCAACGTAA | 0.0639677 | -1 |
| 155 | IGHV1.3 | GTTTCCTGCAAGGCTTCTGGATACACCTTCACTAGCTATGCTATGCATTGGGTGCGCCAG | 0.0624734 | 1 |
| 156 | ECSCR | TGTAAATTATTTTATTGTTTCATGTCTGCTTCTAGATCTAAAGGACACTAGCATTGCCCC | 0.0621209 | 1 |
| 157 | GIMAP4 | AGTGGAGAGTAAGGATGGGATACTTGAATTAATCATGACAGCGTTACAGATTGCTTCCTT | 0.0620186 | 1 |
| 158 | CCR2 | ATGAAGTCATGCGTTTAATCACATTCGAGTGTTTCAGTGCTTCGCAGATGTCCTTGATGC | 0.0612836 | 1 |
| 159 | ABI3BP | CCCACCATAAATGTTACAATATCTGAATATGCTTTGTCAAACTATCCCTTTATGCAATCG | 0.0610981 | 1 |
| 160 | CTSC | AGTCGAAAAATCCCAAGGCCCAAACCTGCACCACTGACTGCTGAAATACAGCAAAAGATT | 0.0610043 | 1 |
| 161 | A_24_P204374 | AGCCTGATGCCTGAACAGTGGAGATCCCGCAGCAGCTACAACTGCTGGGCCATGCATAAA | 0.0608762 | 1 |
| 162 | COL4A4 | GATGCCTGGTCTGCCATTGGCACTTAAATATTTGTTGTATTACGATTTATAATATGGTTC | 0.0606251 | 1 |
| 163 | FGL2 | TTTAATTATCACATCTATTCTTCAATGTGGAAATATTAAATATTGTTGGTTGTAAAATAA | 0.0602213 | 1 |
| 164 | FCRL5 | TGGCATATGGACTGAAAGAAACTATGCTATTGGATCTCCTGGATCTCCAGCTTGCTGACT | 0.0600862 | 1 |
| 165 | SLAIN1 | GCTCTAGGGATATATCAGCAAAAACACATCATGCAATTTGAGACACATAATTTTGTGTTG | 0.0598595 | 1 |
| 166 | RGL1 | TTTGGTTAAACTAATAAACTAGTTTGGGACTTGGCTGGCATGTGCTGCCAGACCCAAAGG | 0.0591349 | 1 |
| 167 | TRABD2A | GACCCCTCGGACTTGAAGAATGGCCATTCCTGTACTCCACATTCTGGTCTAGCCTTGTTG | 0.0590949 | 1 |
| 168 | GCSAM | TATCCTGTGAAAGCATTATCTGTCCCAAACAGTATATGCCTTTTATCAGGGACACATTAA | 0.0590305 | 1 |
| 169 | SRL | ACCTTTCCAGTGTTCTTTTGGTTCTGATTTCATCAGTCTCAATAAAGTTCCGATCTCTCT | 0.0588684 | 1 |
| 170 | CFH | AAGTTTGTACAGGGTAAATCTATAGACGTTGCCTGCCATCCTGGCTACGCTCTTCCAAAA | 0.0574567 | 1 |
| 171 | PROC | CTTCTGGAGGGAAGTAACATTTACTGAGCACCTGTTGTATGTCACATGCCTTATGAATAG | 0.0571625 | -1 |
| 172 | RPLP0P2 | ATGAAGCCAAGGAAGAGTCGGAGGGAGGAGTCAGACGAGGATATGGGATTGGGTCTCTTT | 0.0570625 | -1 |
| 173 | XCL2 | CCAGTTAGCAGAATCAAGACCTACACCATCACGGAAGGCTCCTTGAGAGCAGTAATTTTT | 0.0562159 | 1 |
| 174 | PLA2G4A | GAAATGGCAGCAGTTTCTGATGCTGAGGCAGTTTGCAATCCCATGACAACTGGATTTAAA | 0.0561175 | 1 |
| 175 | NPR1 | TGGAGTGGTGGGACTGAAGATGCCCCGTTACTGTCTCTTTGGGGATACAGTCAACACAGC | 0.0552253 | 1 |
| 176 | TPO | ACAAGTGTGGCTTCCCAGAGAGCGTGGAGAATGGGGACTTTGTGCACTGTGAGGAGTCTG | 0.0551086 | 1 |
| 177 | EMCN | AGTTTTCCGGGCCAAGAATTTTTATCCATGAAGACTTTCCTACTTTTCTCGGTGTTCTTA | 0.0550662 | 1 |
| 178 | LDLRAD4 | AAAGATTTCCACCTGTCACGTATCTGCCTCTTTTACCTGAGCAATGGTGT AGTTCTTAG A | 0.0543361 | |
| 179 | TSPAN32 | ACATTGTCTCCACTCCGAAGCAGTTGCTATTGGTCCAAGAGGATGCTCGGGTAGTCTTCG | 0.0542411 | 1 |
| 180 | TSPAN8 | GCTGATTGGGGAAATAATTTTCAACACTATCCTGAATTATGTGCCTGTCTAGATAAGCAG | 0.0540566 | 1 |
| 181 | GMFG | CTCCAAGAAAAGTTGTCTTTCTTTCGTTGATCTCTGGGCTGGGGACTGAATTCCTGATGT | 0.0540509 | 1 |
| 182 | AK125361 | ACATTCTGAAATCATTTTCTCTGTAAATGGTTGGATTTCATTTCACCCTTAAAGGGATGC | 0.0536025 | 1 |
| 183 | SPRY1 | CTCAAGTTGATATTTATAGCTGATCAATCTATATGTGTCACAGAACTATGCTGCCTAAAG | 0.0533584 | 1 |
| 184 | FAM150B | GTTGACAGTTTGCAGATATATATTCGATAAATCAGTGTACTTGACAGTGTTATCTGTCAC | 0.0533481 | 1 |
| 185 | BMP6 | TGGTTCTCTGCCTTTTTACTATACAGCATACCACGCCACAGGGTTAGAACCAACGAAGAA | 0.0533114 | 1 |
| 186 | CYYR1 | TTGTATTAAGTGTCATAATAGCATCAATGTGCACATTACCAGTATAGACTGTTGGAGTTC | 0.0527333 | 1 |
| 187 | HAAO | GCAGACTCAGTGCAGCACTTCCACACCAAGAAGGCCCTCAATAAAGGCTTCCTGAGGAAC | 0.0526259 | 1 |
| 188 | SH2D3C | GCACAGTGGCACACCACGGAGGCCTGTACCACACCAATGCTGAAGTCAAGCTGCAGGGGT | 0.0525463 | 1 |
| 189 | TSPAN7 | TGTCCCGGTTCATCACGGCCAATCAGTATGAGATGGTGTAAGGAGAAGTCTTTCAAGAAT | 0.0521499 | 1 |
| 190 | AQP1 | CCTCTGCATATATGTCTCTTTGGAGTTGGAATTTCATTATATGTTAAGAAAATAAAGGAA | 0.0516324 | 1 |
| 191 | CD27 | AGCCACAACTGCAGTCCCATCCTCTTGTCAGGGCCCTTTCCTGTGTACACGTGACAGAGT | 0.0510489 | 1 |
| 192 | KCNN3 | AGTGACCAAGCCAACACTCTGGTGGACCTTTCCAAGATGCAGAATGTCATGTATGACTTA | 0.050407 | 1 |
| 193 | CSGALNACT1 | AGCAGTTCTACTCGATCACCAAGATGCTTCTGAAAATTGCATTTTATTACCATTTCAAAC | 0.0503427 | 1 |
| 194 | LGI4 | AAAGCACCGAGGGGTTGATGGCATTCCTTTCCCATTCTAAACTCCACGCAGCCAATCGCT | 0.0499844 | 1 |
| 195 | DMD | GCCTTACTATTGTATTATAGTACTGCTTTACTGTGTATCTCAATAAAGCACGCAGTTATG | 0.0493207 | 1 |
| 196 | PRKACB | GCACCTTAGAAGAATGACACCAGAAAACCTTATTATATCACAATATTCTGTTTTCCCCTT | 0.0490642 | 1 |
| 197 | ATP1B2 | CAGCCTGAAAATTCCAAATCTAGCCTCTGAATGTCTTGGCTCCATCTCTTCAGACCCCTT | 0.0489863 | 1 |
| 198 | TAP2 | GCTCAGTATTTTCGCAAACAGCAGCTGTCTAAGTTCACTGTTCTGAACTTTATTTTTTAA | 0.0489432 | 1 |
| 199 | WNT10B | TTTGGAGCCATAAGATCTGTATCTAGAAAGAGATCACCCACTCCTATGTACTATCCCCAA | 0.0487274 | 1 |
| 200 | ALDH1A1 | TGTCAAACCAGCAGAGCAAACTCCTCTCACTGCTCTCCACGTGGCATCTTTAATAAAAGA | 0.0485829 | 1 |
| 201 | GFI1 | CTGTGGACAAGATGTCATTCATTCACTCAGCAAATGTTCATGGATCACCGGCTACCAAGT | 0.0485136 | 1 |
| 202 | LOC439949 | GCTTGGAGGAAACCAGTTTGCACTATTTGATGAGGAATTTGGCCACCAAACCACTGATAC | 0.0480733 | 1 |
| 203 | KIF19 | GGGAGGGAATCTGGTTTTGTTACTTGGCAGTGGTTTTTTCTCACCCTTCCTTTTTAACAA | 0.0480214 | 1 |
| 204 | PRKCB | CCTCACTTTGATGTTGTTTTGCAAGATGTTTGTGGAAATGTTCATTTGTATCTGGATCTC | 0.0473384 | 1 |
| 205 | RAPH1 | TCATATATCAGGCTATGCAACGTTGCGGAGAGGACCCCCTCCTGCTCCCCCCAAAAGAGA | 0.0472965 | 1 |
| 206 | FNBP1 | TCATTGCAGAAGAAAGTTTACACGACGTCAAAAAGTGACGTTCATGCTAAGTGTTTTTCC | 0.0467515 | 1 |
| 207 | LOC647478 | GATTTTGCAGTGTATTACTGTCAGCAGTATGGTAGCTCACCGTGGACGTTCGGCCAAGGG | 0.0464095 | 1 |
| 208 | PID1 | TGCTCCTTCCTAAAAGTCTGTTTTCAATCCTGGTAATATTAGGGGCACTGCGGCACCTAA | 0.0461036 | 1 |
| 209 | MAP3K14 | CAGGACTCACGTAGCATTAAATCAGCTGTGAATCGTCAGGGGGTGTCTGCTAGCCTCAAC | 0.0457777 | 1 |
| 210 | CD40LG | CTATTGTATCTACCTGCAGTCTCCATTGTTTCCAGAGTGAACTTGTAATTATCTTGTTAT | 0.045739 | 1 |
| 211 | MMRN2 | AACTCTTCACCACACCCTGTATTCTACAACTTCTTTGGTGTTTTGCTCCTCCTGTGGTTG | 0.0455875 | 1 |
| 212 | VWF | TGTACCATGAGGTTCTCAATGCCATGGAGTGCAAATGCTCCCCCAGGAAGTGCAGCAAGT | 0.0455559 | 1 |
| 213 | SLMO1 | CAAAGCCTTCCAGAAGTTTTCTGTGCTTATGAAAGCGTATGTGTGGCCTTTCTCTTCCCT | 0.0452878 | -1 |
| 214 | PI16 | AGGGACGAGGGAAGGAAAGTAACTCCTGACTCTCCAATAAAAACCTGTCCAACCTGTGGC | 0.0452015 | 1 |
| 215 | TOX | TTGGAAACCAAGAGAATGGCTGATGTCTCTTTCTTGGAATATGTGAAATAAATTTAGCAG | 0.0449719 | 1 |
| 216 | JSRP1 | GAGAGGCCTCGGAGAGAGGGGAAGCCGCGGAAGGAGAAGCCGCGGAAGGAGGAGAGACCT | 0.0447145 | 1 |
| 217 | CXorf36 | TCTTGAGGCCCCTGAGAACGTGTGACTCCAGATTTGCCTATCGTTACCCAGATTGCAAAT | 0.044478 | 1 |
| 218 | MISP | TCTTCCTTTAGAAATGTTTCTGCCTTTGGGGTCTAAAGCTTTTGGGGATGAAATGGGACC | 0.0444676 | -1 |
| 219 | COX6C | CATGAAAGATTTTGAGGAGATGAGGAAGGCTGGTATCTTTCAGAGTGTAAAGTAATCTTG | 0.0443444 | -1 |
| 220 | ID4 | AAAGCCACCGGAGGAAAGGAAAAAACATCGGCCAACCTAGAAACGTTTTCATTCGTCATT | 0.0440414 | 1 |
| 221 | ATOH8 | GGGCAGGCCCTGCCATGGACAGAAAAAGAGTTTTTCTCTTGTTCAGCCTGCACGTGGCCT | 0.0438109 | 1 |
| 222 | CHRDL1 | TATATGTGGGAGGAGGAGGGATAGAACATCACAACACTGCTCTAGTTTCTTGGAGAATCA | 0.0436953 | 1 |
| 223 | B3GAT1 | ACTTCTTTGGGCAGATGCTAGGTCAGTTGTTTTCACCTAATATCCTCTTTTAGCTGCATG | 0.0435041 | 1 |
| 224 | S1PR1 | GCTGAGGCCAAAGTTTCCATGTAAGCGGGATCCGTTTTTTGGAATTTGGTTGAAGTCACT | 0.0432785 | 1 |
| 225 | CCM2L | CTTATATGACCTCAAACTGACCATACTAAACAGTGTAGAAGGTCTTTTTAAGGCTCTAAA | 0.0432204 | 1 |
| 226 | GPR20 | ACTGAAAACTCACAGGGGTAGCCGGGAGCTGCTCCTCACTTTGGCCAGTTTGTCTCCAGA | 0.0428173 | 1 |
| 227 | TNFRSF8 | GAGGGATTGACATGTTTCAACAAAATAATGCACTTCCTTACCTAGTGGCCCTTCACACAA | 0.0426749 | 1 |
| 228 | LOC728392 | CCTGCATATTATTTACTTGCAAATGTCTGTAATCTGTAATTGTGATGCCTCTGATGGAAT | 0.0418649 | 1 |
| 229 | AMICA1 | CTCCTGTGGGCAGGGTTCTTAGTGGATGAGTTACTGGGAAGAATCAGAGATAAAAACCAA | 0.0416444 | 1 |
| 230 | PHYHIP | GTGACATCAGGCAGAGGAGAGTGCAGCCTCACAGTGACTTTCTCAGAGGTGACAGAGATG | 0.041561 | 1 |
| 231 | DUSP26 | CCAAAAACATACAGAGGTGCATGGCTGGCAGTCTTGAAATTGTCACTCGCTTACTGGATC | 0.0410888 | 1 |
| 232 | A_24_P76868 | TGGGCATCACCGGACTCCAGACTGGGGACGAGGCCGATTATTACTGCGGAACATGGAATA | 0.0410874 | 1 |
| 233 | NTM | CTCTTGGTTTATTTTATTTCCCACCTTTGTGTGAGTGTGTATGAAAGAGAAGAAAATGCA | 0.0407045 | -1 |
| 234 | IL18RAP | ACAGAACTCACAGCTCTGTGTGTGTGTGTTCAGGCTGATAGGAAATTCAAAGAGTCTCCT | 0.0404772 | 1 |
| 235 | SPP1 | TTCCACAGCCATGAATTTCACAGCCATGAAGATATGCTGGTTGTAGACCCCAAAAGTAAG | 0.0400487 | -1 |
| 236 | IL3RA | GAGATGCCTGTGTAATTTCGTCCGAAGCTGCCAGGAAGAAGAACAGAACTTTGTGTGTTT | 0.0397441 | 1 |
| 237 | PSAT1 | TACCATTCTTTCCATAGGTAGAAGAGAAAGTTGATTGGTTGGTTGTTTTTCAATTATGCC | 0.0396759 | 1 |
| 238 | RASGRP2 | GGCCAGGGCTGGTGTCCCTAAGGTTGTACAGACTCTTGTGAATATTTGTATTTTCCAGAT | 0.0393138 | 1 |
| 239 | FAM117A | GACTCTATTTTCTCCCAAAGACACTATTTTTGCAGCTGTTTGAAGTTTGTATATTTTCCG | 0.0392651 | 1 |
| 240 | CCL19 | AGTGTGAGTGTGAGCGAGAGGGTGAGTGTGGTCAGAGTAAAGCTGCTCCACCCCCAGATT | 0.0389893 | 1 |
| 241 | ST6GALNAC1 | TGATGGGTGGCCAATACCACAATTCCTGCTGAAAAACACTCTTCCAGTCCAAAAGCTTCT | 0.0389546 | 1 |
| 242 | FAM162B | CCCAAACATAGCAGTTTTTCTGAGTTTCATCACCTTTGATTCATTTTGCCTGTTTTTGAA | 0.038948 | 1 |
| 243 | TMOD1 | GGGCAAATGGCTTATGTGAGGTAAGACACTAGAGGGATAAATTTCCAGATCAACATGGCT | 0.0388902 | 1 |
| 244 | ESM1 | CAGAGGGACCTTATTTAAACATAAGTGCTGTGACTTCGGTGAATTTTCAATTTAAGGTAT | 0.0388171 | -1 |
| 245 | FXYD1 | ATGGCGTCTCTTGGCCACATCTTGGTTTTCTGTGTGGGTCTCCTCACCATGGCCAAGGCA | 0.0385533 | 1 |
| 246 | LOC572558 | TTTTTCTGTGAAGTCAAGAAAGTGGTTACAATGGTACTTTCAGCCTGTCCGAATTATGTA | 0.0383883 | 1 |
| 247 | KANK3 | GACAATGGAGAGAACCCCCAGGTTCAGTAAGCTGCCTCGTCTGGCTCACTACACCTAGCT | 0.0383789 | 1 |
| 248 | BTLA | GTCAGAGACATACATGCATTTTCCCAAATGACTCTACTTCACTATTATTTACATGGCTTA | 0.0383016 | 1 |
| 249 | FAM13A | GTTTGGAATTCTTTGCTCTACTGTTTACATTGCAGATTGCTATAATTTCAAGGAGTGAGA | 0.0381754 | 1 |
| 250 | TTN | CTGACAACCCTGATCATCATGGACGTACAGAAACAAGATGGTGGACTTTATACCCTGAGT | 0.0381347 | 1 |
| 251 | AGPAT9 | GTAACAAAGAGCTTAGTTTTCCTTGTTTGAATGCTGTAGATCTGTACCTAGTACCCCTCC | 0.0374138 | 1 |
| 252 | MMRN1 | GGTGAACATGCTTAAAAATACATTCCTTGGATATCTGACAACGTGTTTCTGAAAAACAGA | 0.0367607 | 1 |
| 253 | BCL11B | AAACCCGTGATTTTGGTGCTCCTTGTAACTCAGCCCTGCAAAGCAAAGTCCCATTGATTT | 0.0363941 | 1 |
| 254 | CHL1 | CAAACTCTCTCAACTCTAAAGTGCTAATAATAATCTCAGTTACCTTATCTTTGTCACAGG | 0.0361686 | 1 |
| 255 | SULF2 | AACCTAATAAGAAATCCCAATTTTCAGGAGTGGTGGTGTCAATAAACGCTCTGTGGCCAG | 0.0361556 | -1 |
| 256 | SLCO4A1 | TATTTATGGACACACAGTTTGCATCAGAACGTGTTTATAGAATGTGTTTTATACCCGATC | 0.0361444 | 1 |
| 257 | LOC283867 | CTCTGTTGGTCCGACTTACATGAAACAAGAGAGTTATTTTGTTGTTGTTGTTTTGTTTTG | 0.0352232 | -1 |
| 258 | CCL21 | AAGGAGCTCTGGGTGCAGCAGCTGATGCAGCATCTGGACAAGACACCATCCCCACAGAAA | 0.0351541 | 1 |
| 259 | FAM135B | TTTAACTTCAATATGCTGTCTGGACATAGTGAGAACTAATGGCAATAAACGTCCAGCTGC | 0.0348572 | -1 |
| 260 | CYTIP | TTCATCGTGCTGTGGAAGAGGAAGAAAGTCGCTTTTGACGGATTGTGGTGTCCTTTCAAA | 0.0347307 | 1 |
| 261 | IGHA2 | GTAAACCCACCCACATCAATGTGTCTGTTGTCATGGCGGAGGCGGATGGCACCTGCTACT | 0.0346468 | 1 |
| 262 | FCRL6 | AAAGGGAAAGATGAAGGTGTTGTCTACTCTGTGGTGCATAGAACCTCAAAGAGGAGTGAA | 0.0344813 | 1 |
| 263 | CCDC178 | AAGAGAGAATAATTACTGGCGGTGTTGACAGTGACTGTTCGCTTCCCCAGATTTCCCTAT | 0.0343785 | 1 |
| 264 | THEMIS | CTGAAATTTCACCAGATTTCTGAGACGATGTCTTAATATTCTATGTGCTATGTACCAGAT | 0.0343529 | 1 |
| 265 | CCND1 | ACATTGCCAGGATGATAAGTTCCTTTCCTTTTCTTTAAAGAAGTTGAAGTTTAGGAATCC | 0.0340949 | -1 |
| 266 | PRKCH | TGAATGGTGTTTGCATTCTGTGAAATGCCTCTCCACGTTGCATATGTCACACTTTTGTCT | 0.0338977 | 1 |
| 267 | CLEC10A | AGGACTCTTCTCACGACCTCCTCGCAAGACCGCTCTGGGAGAGAAATAAGCACTGGGAGA | 0.0337595 | 1 |
| 268 | IGFN1 | ATGGAGGCTGTGCCCAGAGCCCCAGGAAAATGGGAGTGAGAAGATGCCTGGCGAGGTTTT | 0.0336311 | 1 |
| 269 | ITIH4 | GCCGCTTCTGGGGCCTGGACCACCATGGGGAGGAAGAGTCCCACTCATTACAAATAAAGA | 0.0333221 | 1 |
| 270 | ZEB2 | CTTTTAATCTGTGTTTCTGCAAGTGCCATCCTTGTACAGTGTTAAGAGGGTAACATGGGT | 0.0330172 | 1 |
| 271 | TLE4 | ACCTCCCTCTGAAAAACACAAAGAATGGACTCTCTCCTGGGATGAGGACTTGCTTTCTTT | 0.0328493 | 1 |
| 272 | LOC339524 | TACTAGGGGATCCTCTTTTAAATATGGAGGCCCAAATCAGAAGCTTGTAGAGGGGAGCTA | 0.0328183 | 1 |
| 273 | KCNA3 | ATCCCCTTTCCCGGAATTTTAAGTGTCTCTACAACTTTGAATAAAGGGAAATGCCCAAGA | 0.0327574 | 1 |
| 274 | ENST00000445121 | CTGAAGATTGAGCTCCCAACCCCCAAGTACGAAATAGGCTAAACCAATAAAAAATTGTGT | 0.0326714 | 1 |
| 275 | ABLIM1 | AAGCAAAACTCTTCTAAGTCCCACTCGTGAATGGCAATTAGAGAAAGGACTGACAGTGGC | 0.0322961 | 1 |
| 276 | CARD16 | ACCAGGAAGAGATGGAGAAAGTAAAACGTGAAAATGCTACAGTTATGGATAAGACCCGAG | 0.0322958 | 1 |
| 277 | EBF3 | AAATTGCCTCCATTTTATTTTGTACGAGTAAGGGTTTGATCTACAAAAGAGCTCACATGG | 0.0321831 | 1 |
| 278 | PPEF1 | GGAAGTCCCTAGCAAGCTGTTATTGGTAAGATTAGGTTAAATGTCAGTAATAGGATTTGG | 0.0320775 | -1 |
| 279 | GPR133 | GAACTATCTTTAGTTTAGATGGAATTATTTGTTTTTAATTGTTGCCGTATTCATCTATAT | 0.0320379 | 1 |
| 280 | ELOVL6 | TGACAACAAAGGGCCCCCATACACTTATCCCTCAAATTTTAAGTGATATGAAATACTTGT | 0.0319714 | -1 |
| 281 | MICAL2 | CACTGTATTTGTGCAGATCCTGGCCAGTACAAAGTCGTTGCTCTTGTCTTATCTTCTCTT | 0.0316668 | -1 |
| 282 | MT1JP | TGCGCCTTGCGGTCTCTCCATTTATCGCTTGAGATCTCCAGCCTTACCGCGGCTCGAAAT | 0.0316355 | 1 |
| 283 | HOXA5 | ACCCGCAGAAGGAGGATTGAAATAGCACATGCTCTTTGCCTCTCCGAGAGACAAATTAAA | 0.0314651 | 1 |
| 284 | SMR3A | CACCCTATGGTCCAGGGAGAATTCAATCACACTCTCTTCCTCCTCCTTATGGCCCAGGTT | 0.0314615 | 1 |
| 285 | PPAP2B | TATGGATCAACCACATGCACATCCTTACTACAGAATCCGTCCTTTCATTTCAACTTATAG | 0.0311292 | 1 |
| 286 | GRM8 | AAGACAATGAGTCTGTTTCTTGTAATGGCTGACCAGATTGAAGCCCTGGGTTGTGCTAAA | 0.0308422 | -1 |
| 287 | PALM2 | GCATGAGTCACAATTACAAAGTTTTGAGCGGTTTTGTAATTTGACATTTAGGAAAGTCTC | 0.0306482 | 1 |
| 288 | ICAM4 | AATAAAAATAAATATTGGCGGGGGAACCCTCTGGAATCAATAAAGGCTTCCTTAACCAGC | 0.0306111 | 1 |
| 289 | DLG5 | AGTTTTCTAGCTCAGACAAACTATGAAAAACTGTAGACTATTCTCAAGGTATTAACTCGC | 0.0303506 | -1 |
| 290 | EBF1 | ACCAGCAGATTGGATGCCTTAACAATGCAAATCATATTCATTTCACTTGTACATTGTAAC | 0.030188 | 1 |
| 291 | FOXN2 | AGTCACAAGTGAACCACTTCTCAGTTGCCAATCTTTGCTCATATTAAAAACAACTTACAA | 0.0301556 | 1 |
| 292 | TMEM100 | GACTGAAAAATCAGCTTTCTATTTACATGAAACACTTTGGGGGTCATGGGAGTGCACAGC | 0.0300854 | 1 |
| 293 | ANGPT1 | GTTTCAAACTTGGTGACTTGATCCACTATGCCTTAATGGTTTCCTCCATTTGAGAAAATA | 0.029906 | 1 |
| 294 | IGKV6.21 | CAATAGCCTGGAAGCTGAAGATGCTGCAACGTATTACTGTCATCAGAGTAGTAGTTTACC | 0.029898 | 1 |
| 295 | LOC643431 | GTAGAGCTGGAGTCTCGCCTGGAAGGGCTGACTGAGATCAACTTCCTCAGGCAGCTGTAT | 0.0298316 | -1 |
| 296 | PRDM16 | CTAGGAGAAGCCGATTTGGAGGTTAATGCTGTAGAATAGGACTGTATACCAAATGTAATC | 0.0297239 | 1 |

## Claims

1. A method of typing a sample from an invasive lobular breast cancer patient, the method comprising:
providing a sample comprising RNA expression products from breast cancer cells from the patient;
determining a level of RNA expression for at least two genes that are selected from Table 1 comprising at least GIMAP6 and P2RY14 in said sample;
comparing said determined level of RNA expression of the at least two genes to the level of expression of the genes in a reference sample; and
typing said sample based on the comparison of the determined levels of RNA expression.

2. The method according to claim 1, wherein the reference sample comprises a pooled RNA sample that is isolated from tissue comprising breast cancer cells from multiple individuals suffering from breast cancer.

3. The method according to claim 1 or claim 2, further comprising determining a level of expression of PDL1, PD1, and/or CTLA4.

4. The method according to any one of the previous claims, whereby a level of RNA expression of at least five genes from Table 1, comprising at least GIMAP6 and P2RY14, is determined.

5. The method according to any one of the previous claims, whereby a level of RNA expression of at least GIMAP6, P2RY14, CD79B, GIMAP5, GIMAP8, LOC100290115, IL33, SEL1L3, and SLC9A9 is determined

6. The method according to any one of the previous claims, whereby a level of RNA expression of all genes from Table 1 is determined.

7. The method according to any of claims 1-6, further comprising determining a metastasizing potential of the sample from the patient.

8. A method of assigning treatment to a patient suffering from breast cancer, comprising
(a) typing a sample comprising RNA expression products from breast cancer cells from the patient according to the method of any of claims 1-7;
(b) classifying said sample as Immune Related (IR) or Hormone Response (HR);
(c) assigning anti-Estrogen Receptor directed therapy to a patient of which the sample is classified as HR.

9. A method of assigning treatment to a patient suffering from breast cancer, comprising
(a) typing a sample comprising RNA expression products from breast cancer cells from the patient according to the method of any of claims 1-7;
(b) classifying said sample as Immune Related (IR) or Hormone Response (HR);
(c) assigning therapy targeting PD1, PDL1 and/or CTLA4 and/or assigning therapy comprising at least one DNA damaging agent to a patient of which the sample is classified as IR.

10. The method according to claim 9, whereby the therapy comprises antibodies to PD1, PDL1 and/or CTLA4.

11. The method according to claim 9 or claim 10, whereby the DNA damaging agent comprises bleomycin, cisplatin and/or a topoisomerase 1 inhibitor.

12. The method according to any one of claims 9-11, wherein the DNA damaging agent comprises topoisomerase 1 inhibitor SN-38.

13. Anti-Estrogen Receptor directed therapy for use in a method of treating a patient suffering from breast cancer, comprising
(a) typing a sample comprising RNA expression products from breast cancer cells from the patient according to the method of any of claims 1-7;
(b) classifying said sample as Immune Related (IR) or Hormone Response (HR);
(c) treating a patient of which the sample is classified as HR with anti-Estrogen Receptor directed therapy.

14. PD1, PDL1 and/or CTLA4-targeting therapy, and/or a therapy comprising at least one DNA damaging agent, for use in a method of treating a patient suffering from breast cancer, comprising
(a) typing a sample comprising RNA expression products from breast cancer cells from the patient according to the method of any of claims 1-7;
(b) classifying said sample as Immune Related (IR) or Hormone Response (HR);
(c) treating a patient of which the sample is classified as IR with therapy targeting PD1, PDL1 and/or CTLA4 and/or with therapy comprising at least one DNA damaging agent.

## Patentansprüche

1. Verfahren zum Typisieren einer Probe von einem invasiv lobulären Brustkrebs Patienten, das Verfahren umfassend:
Bereitstellen einer Probe, umfassend RNA-Expressionsprodukte von Brustkrebszellen von dem Patienten;
Bestimmen eines Grades der RNA-Expression für wenigstens zwei Gene, die ausgewählt sind aus Tabelle 1, umfassend wenigstens GIMAP6 und P2RY14, in der Probe;
Vergleichen des bestimmten Grades der RNA-Expression von den wenigstens zwei Genen mit dem Grad der Expression der Gene in einer Referenzprobe; und
Typisieren der Probe basierend auf dem Vergleich der bestimmten Grade der RNA-Expression.

2. Verfahren nach Anspruch 1, wobei die Referenzprobe eine vereinigte RNA-Probe umfasst, die aus Gewebe isoliert wird, umfassend Brustkrebszellen von mehreren Individuen, leidend an Brustkrebs.

3. Verfahren nach Anspruch 1 oder Anspruch 2, ferner umfassend Bestimmen eines Grades der Expression von PDL1, PD1 und/oder CTLA4.

4. Verfahren nach einem der vorhergehenden Ansprüche, wonach ein Grad der RNA-Expression von wenigstens fünf Genen aus Tabelle 1, umfassend wenigstens GIMAP6 und P2RY14, bestimmt wird.

5. Verfahren nach einem der vorhergehenden Ansprüche, wonach ein Grad der RNA-Expression von wenigstens GIMAP6, P2RY14, CD79B, GIMAP5, GIMAP8, LOC100290115, IL33, SEL1L3 und SLC9A9 bestimmt wird.

6. Verfahren nach einem der vorhergehenden Ansprüche, wonach ein Grad der RNA-Expression aller Gene aus Tabelle 1 bestimmt wird.

7. Verfahren nach einem der Ansprüche 1-6, ferner umfassend Bestimmen eines Metastasierungspotenzials der Probe von der Patientin.

8. Verfahren zum Zuweisen von Behandlung für einen Patienten, leidend an Brustkrebs, umfassend
(a) Typisieren einer Probe, umfassend RNA-Expressionsprodukte aus Brustkrebszellen von dem Patienten nach dem Verfahren von einem der Ansprüche 1-7;
(b) Klassifizieren der Probe als Immunbezogen (IR) oder Hormonantwort (HR);
(c) Zuweisen von Anti-Östrogenrezeptor gerichteter Therapie für einen Patienten, dessen Probe als HR klassifiziert wird.

9. Verfahren zum Zuweisen von Behandlung für einen Patienten, leidend an Brustkrebs, umfassend
(a) Typisieren einer Probe, umfassend RNA-Expressionsprodukte aus Brustkrebszellen von dem Patientennach dem Verfahren von einem der Ansprüche 1-7;
(b) Klassifizieren der Probe als Immunbezogen (IR) oder Hormonantwort (HR);
(c) Zuweisen von Therapie, zielend auf PD1, PDL1 und/oder CTLA4 und/oder Zuweisen von Therapie, umfassend wenigstens einen DNA-schädigenden Wirkstoff, für einen Patienten, dessen Probe als IR klassifiziert wird.

10. Verfahren nach Anspruch 9, wonach die Therapie Antikörper für PD1, PDL1 und/oder CTLA4 umfasst.

11. Verfahren nach Anspruch 9 oder Anspruch 10, wonach der DNAschädigende Wirkstoff Bleomycin, Cisplatin und/oder einen Topoisomerase-1-Inhibitor umfasst.

12. Verfahren nach einem der Ansprüche 9-11, wobei der DNAschädigende Wirkstoff Topoisomerase-1-Inhibitor SN-38 umfasst.

13. Anti-Östrogenrezeptor gerichtete Therapie zur Verwendung in einem Verfahren zum Behandeln eines Patienten, leidend an Brustkrebs, umfassend:
(a) Typisieren einer Probe, umfassend RNA-Expressionsprodukte aus Brustkrebszellen von dem Patienten nach dem Verfahren von einem der Ansprüche 1-7;
(b) Klassifizieren der Probe als Immunbezogen (IR) oder Hormonantwort (HR);
(c) Behandeln eines Patienten, dessen Probe als HR klassifiziert wird, mit Anti-Östrogenrezeptor gerichteter Therapie.

14. Auf PD1, PDL1 und/oder CTLA4 zielende Therapie und/oder Therapie, umfassend wenigstens einen DNA-schädigenden Wirkstoff, zur Verwendung in einem Verfahren zum Behandeln eines Patienten, leidend an Brustkrebs, umfassend:
(a) Typisieren einer Probe, umfassend RNA-Expressionsprodukte aus Brustkrebszellen von dem Patienten nach dem Verfahren von einem der Ansprüche 1-7;
(b) Klassifizieren der Probe als Immunbezogen (IR) oder Hormonantwort (HR);
(c) Behandeln eines Patienten, dessen Probe als IR klassifiziert wird, mit Therapie, zielend auf PD1, PDL1 und/oder CTLA4 und/oder mit Therapie, umfassend wenigstens einen DNA-schädigenden Wirkstoff.

## Revendications

1. Procédé de typage d'un échantillon provenant d'un(e) patient(e) souffrant d'un cancer du sein lobulaire invasif, le procédé comprenant les étapes consistant à :
fournir un échantillon comprenant des produits d'expression d'ARN provenant de cellules cancéreuses mammaires du/de la patient(e) ;
déterminer un taux d'expression d'ARN pour au moins deux gènes qui sont choisis dans le tableau 1, comprenant au moins GIMAP6 et P2RY14 dans ledit échantillon ;
comparer ledit taux déterminé d'expression d'ARN desdits au moins deux gènes au taux d'expression des gènes dans un échantillon de référence ; et
typer ledit échantillon sur la base de la comparaison des taux déterminés d'expression d'ARN.

2. Procédé selon la revendication 1, dans lequel l'échantillon de référence comprend un échantillon d'ARN groupés qui est isolé à partir d'un tissu comprenant des cellules cancéreuses mammaires provenant de multiples individus souffrant de cancer du sein.

3. Procédé selon la revendication 1 ou la revendication 2, comprenant encore le fait de déterminer un taux d'expression de PDL1, PD1, et/ou CTLA4.

4. Procédé selon l'une quelconque des revendications précédentes, dans lequel on détermine un taux d'expression d'ARN d'au moins cinq gènes du tableau 1, comprenant au moins GIMAP6 et P2RY14.

5. Procédé selon l'une quelconque des revendications précédentes, dans lequel on détermine un taux d'expression d'ARN d'au moins GIMAP6, P2RY14, CD79B, GIMAP5, GIMAP8, LOC100290115, IL33, SEL1L3, et SLC9A9.

6. Procédé selon l'une quelconque des revendications précédentes, dans lequel on détermine un taux d'expression d'ARN de tous les gènes du tableau 1.

7. Procédé selon l'une quelconque des revendications 1 à 6, comprenant encore le fait de déterminer un potentiel de dissémination de métastases de l'échantillon provenant du/de la patient(e).

8. Procédé d'attribution d'un traitement à un(e) patient(e) souffrant d'un cancer du sein, comprenant les étapes consistant à :
(a) typer un échantillon comprenant des produits d'expression d'ARN provenant de cellules cancéreuses mammaires du/de la patient(e) conformément au procédé selon l'une quelconque des revendications 1 à 7 ;
(b) classer ledit échantillon en tant qu'immuno-associé (IR) ou à réponse hormonale (HR) ;
(c) attribuer une thérapie dirigée sur anti-récepteurs d'oestrogènes à un(e) patient(e) dont l'échantillon est classé en tant que HR.

9. Procédé d'attribution d'un traitement à un(e) patient(e) souffrant d'un cancer du sein, comprenant les étapes consistant à :
(a) typer un échantillon comprenant des produits d'expression d'ARN provenant de cellules cancéreuses mammaires du/de la patient(e) conformément au procédé selon l'une quelconque des revendications 1 à 7 ;
(b) classer ledit échantillon en tant qu'immuno-associé (IR) ou à réponse hormonale (HR) ;
(c) attribuer une thérapie ciblant PD1, PDL1 et/ou CTLA4 et/ou attribuer une thérapie comprenant au moins un agent endommageant l'ADN à un(e) patient(e) dont l'échantillon est classé en tant que IR.

10. Procédé selon la revendication 9, dans lequel la thérapie comprend des anticorps dirigés contre PD1, PDL1 et/ou CTLA4.

11. Procédé selon la revendication 9 ou la revendication 10, dans lequel l'agent endommageant l'ADN comprend la bléomycine, le cisplatine et/ou un inhibiteur de topoisomérase 1.

12. Procédé selon l'une quelconque des revendications 9 à 11, dans lequel l'agent endommageant l'ADN comprend l'inhibiteur de topoisomérase 1 SN-38.

13. Thérapie dirigée sur anti-récepteurs d'oestrogènes, destinée à être utilisée dans un procédé de traitement d'un(e) patient(e) souffrant de cancer du sein, comprenant
(a) le typage d'un échantillon comprenant des produits d'expression d'ARN provenant de cellules cancéreuses mammaires du/de la patient(e) conformément au procédé selon l'une quelconque des revendications 1 à 7 ;
(b) le classement dudit échantillon en tant qu'immuno-associé (IR) ou à réponse hormonale (HR) ;
(c) le traitement d'un(e) patient(e) dont l'échantillon est classé en tant que IR par une thérapie dirigée sur anti-récepteurs d'oestrogènes.

14. Thérapie ciblant PD1, PDL1 et/ou CTLA4, et/ou thérapie comprenant au moins un agent endommageant l'ADN, destinée(s) à être utilisée(s) dans un procédé de traitement d'un(e) patient(e) souffrant de cancer du sein, comprenant
(a) le typage d'un échantillon comprenant des produits d'expression d'ARN provenant de cellules cancéreuses mammaires du/de la patient(e) conformément au procédé selon l'une quelconque des revendications 1 à 7 ;
(b) le classement dudit échantillon en tant qu'immuno-associé (IR) ou à réponse hormonale (HR) ;
(c) le traitement d'un(e) patient(e) dont l'échantillon est classé en tant que IR par une thérapie ciblant PD1, PDL1 et/ou CTLA4, et/ou par une thérapie comprenant au moins un agent endommageant l'ADN.
